# EUROPEAN PATENT APPLICATION

(11) **EP 2 196 218 A2**
(43) Date of publication of application: **16.06.2010**
(21) Application number: 10155524.1
(22) Date of filing: 24.04.2003
(51) Int. Cl.: A61K 39/395, A61K 33/06, A61K 39/295, A61K 31/33, A61K 31/16, A61K 31/56, A61K 31/135, A61K 31/215, A61K 31/167, A61K 31/192, A61K 31/196, A61K 31/415, A61K 31/45, A61K 31/485, A61K 31/519, A61K 31/573, A61K 31/58, C07K 16/24

(54) **Use of anti-TNFalpha antibodies and another drug**

(30) Priority: 26.04.2002 US 133715
(62) Divisional of application: 03762970.6
(71) Applicant: Abbott Biotechnology Ltd, HM 11 Hamilton (BM)
(72) Inventor: Fischkoff, Steven, Short Hills, NJ 07078 (US); Chartash, Elliot, Randolph, NJ 07869 (US)
(74) Representative: Modiano, Micaela Nadia

(57) **Abstract**

The present invention is directed to a method of treating disorders in which TFNalpha activity is detrimental via biweekly, subcutaneous administration of human antibodies, preferably recombinant human antibodies, that specifically bind to human tumor necrosis factor alpha (hTNFalpha) in combination with another drug which is useful for treating the disorder. An antibody of the invention can be a full-length antibody or an antigen-binding portion thereof. Kits containing a pharmaceutical composition and instructions for dosing are also encompassed by the invention.

SEQUENCE LISTING

<110> Fischkoff, Steven
      Chartash, Elliot

<120> Use of TNF-a Antibodies and Another Drug


<130> BBI-186

<140> 10/133,715
<141> 2002-04-26

<160> 37

<170> FastSEQ for Windows Version 4.0

<210> 1
<211> 107
<212> PRT
<213> Artificial Sequence

<220>
<223> D2E7 light chain variable region

<400> 1


<210> 2
<211> 121
<212> PRT
<213> Artificial Sequence

<220>
<223> D2E7 heavy chain variable region

<400> 2

<210> 3
<211> 9
<212> PRT
<213> Artificial Sequence

<220>
<223> D2E7 light chain variable region CDR3

<221> VARIANT
<222>)9
<223> Xaa = Any Amino Acid

<400> 3
Gln Arg Tyr Asn Arg Ala Pro Tyr Xaa
 1               5


<210> 4
<211> 12
<212> PRT
<213> Artificial Sequence

<220>
<223> D2E7 heavy chain variable region CDR3

<221> VARIANT
<222> 12
<223> Xaa = Any Amino Acid

<400> 4
Val Ser Tyr Leu Ser Thr Ala Ser Ser Leu Asp Xaa
 1               5                   10


<210> 5
<211> 7
<212> PRT
<213> Artificial Sequence

<220>
<223> D2E7 light chain variable region CDR2

<400> 5
Ala Ala Ser Thr Leu Gln Ser
 1               5


<210> 6
<211> 17
<212> PRT
<213> Artificial Sequence

<220>
<223> D2E7 heavy chain variable region CDR2

<400> 6
Ala Ile Thr Trp Asn Ser Gly His Ile Asp Tyr Ala Asp Ser Val Glu
 1               5                   10                  15
Gly


<210> 7
<211> 11
<212> PRT
<213> Artificial Sequence

<220>

<223> D2E7 light chain variable region CDR1

<400> 7
Arg Ala Ser Gln Gly Ile Arg Asn Tyr Leu Ala
 1               5                   10


<210> 8
<211> 5
<212> PRT
<213> Artificial Sequence

<220>
<223> D2E7 heavy chain variable region CDR1

<400> 8
Asp Tyr Ala Met His
 1               5


<210> 9
<211> 107
<212> PRT
<213> Artificial Sequence

<220>
<223> 2SD4 light chain variable region

<400> 9
Asp Ile Gln Met Thr Gln Ser Pro Ser Ser Leu Ser Ala Ser Ile Gly
 1               5                   10                  15
Asp Arg Val Thr Ile Thr Cys Arg Ala Ser Gln Gly Ile Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Thr Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Lys Tyr Asn Ser Ala Pro Tyr
                85                  90                  95
Ala Phe Gly Gln Gly Thr Lys Val Glu Ile Lys
            100                 105


<210> 10
<211> 121
<212> PRT
<213> Artificial Sequence

<220>
<223> 2SD4 heavy chain variable region

<400> 10
Gln Val Gln Leu Val Glu Ser Gly Gly Gly Leu Val Gln Pro Gly Arg
 1               5                   10                  15
Ser Leu Arg Leu Ser Cys Ala Ala Ser Gly Phe Thr Phe Asp Asp Tyr
            20                  25                  30
Ala Met His Trp Val Arg Gln Ala Pro Gly Lys Gly Leu Asp Trp Val
        35                  40                  45
Ser Ala Ile Thr Trp Asn Ser Gly His Ile Asp Tyr Ala Asp Ser Val
    50                  55                  60
Glu Gly Arg Phe Ala Val Ser Arg Asp Asn Ala Lys Asn Ala Leu Tyr
65                  70                  75                  80
Leu Gln Met Asn Ser Leu Arg Pro Glu Asp Thr Ala Val Tyr Tyr Cys
                85                  90                  95
Thr Lys Ala Ser Tyr Leu Ser Thr Ser Ser Ser Leu Asp Asn Trp Gly
            100                 105                 110
Gln Gly Thr Leu Val Thr Val Ser Ser

115                 120


<210> 11
<211> 9
<212> PRT
<213> Artificial Sequence

<220>
<223> 2SD4 light chain variable region CDR3

<400> 11
Gln Lys Tyr Asn Ser Ala Pro Tyr Ala
1               5


<210> 12
<211> 9
<212> PRT
<213> Artificial Sequence

<220>
<223> EP B12  light chain variable region CDR3

<400> 12
Gln Lys Tyr Asn Arg Ala Pro Tyr Ala
1               5


<210> 13
<211> 9
<212> PRT
<213> Artificial Sequence

<220>
<223> VL10E4 light chain variable region CDR3

<400> 13
Gln Lys Tyr Gln Arg Ala Pro Tyr Thr
1               5


<210> 14
<211> 9
<212> PRT
<213> Artificial Sequence

<220>
<223> VL100A9 light chain variable region CDR3

<400> 14
Gln Lys Tyr Ser Ser Ala Pro Tyr Thr
1               5


<210> 15
<211> 9
<212> PRT
<213> Artificial Sequence

<220>
<223> VLL100D2 light chain variable region CDR3

<400> 15
Gln Lys Tyr Asn Ser Ala Pro Tyr Thr
1               5


<210> 16

<211> 9
<212> PRT
<213> Artificial Sequence

<220>
<223> VLL0F4 light chain variable region CDR3

<400> 16
Gln Lys Tyr Asn Arg Ala Pro Tyr Thr
1               5


<210> 17
<211> 9
<212> PRT
<213> Artificial Sequence

<220>
<223> L0E5 light chain variable region CDR3

<400> 17
Gln Lys Tyr Asn Ser Ala Pro Tyr Tyr
1               5


<210> 18
<211> 9
<212> PRT
<213> Artificial Sequence

<220>
<223> VLL0G7 light chain variable region CDR3

<400> 18
Gln Lys Tyr Asn Ser Ala Pro Tyr Asn
1               5


<210> 19
<211> 9
<212> PRT
<213> Artificial Sequence

<220>
<223> VLL0G9 light chain variable region CDR3

<400> 19
Gln Lys Tyr Thr Ser Ala Pro Tyr Thr
1               5


<210> 20
<211> 9
<212> PRT
<213> Artificial Sequence

<220>
<223> VLL0H1 light chain variable region CDR3

<400> 20
Gln Lys Tyr Asn Arg Ala Pro Tyr Asn
1               5


<210> 21
<211> 9
<212> PRT
<213> Artificial Sequence

<220>
<223> VLL0H10 light chain variable region CDR3

<400> 21
Gln Lys Tyr Asn Ser Ala Ala Tyr Ser
1               5


<210> 22
<211> 9
<212> PRT
<213> Artificial Sequence

<220>
<223> VL1B7 light chain variable region CDR3

<400> 22
Gln Gln Tyr Asn Ser Ala Pro Asp Thr
1               5


<210> 23
<211> 9
<212> PRT
<213> Artificial Sequence

<220>
<223> VL1C1 light chain variable region CDR3

<400> 23
Gln Lys Tyr Asn Ser Asp Pro Tyr Thr
1               5


<210> 24
<211> 9
<212> PRT
<213> Artificial Sequence

<220>
<223> VL0.1F4 light chain variable region CDR3

<400> 24
Gln Lys Tyr Ile Ser Ala Pro Tyr Thr
1               5


<210> 25
<211> 9
<212> PRT
<213> Artificial Sequence

<220>
<223> VL0.1H8 light chain variable region CDR3

<400> 25
Gln Lys Tyr Asn Arg Pro Pro Tyr Thr
1               5


<210> 26
<211> 9
<212> PRT
<213> Artificial Sequence

<220>
<223> L0E7.A light chain variable region CDR3

<400> 26

Gln Arg Tyr Asn Arg Ala Pro Tyr Ala
1               5

<210> 27
<211> 12
<212> PRT
<213> Artificial Sequence

<220>
<223> 2SD4 heavy chain variable region CDR3

<400> 27
Ala Ser Tyr Leu Ser Thr Ser Ser Ser Leu Asp Asn
1               5                   10

<210> 28
<211> 12
<212> PRT
<213> Artificial Sequence

<220>
<223> VH1B11 heavy chain variable region CDR3

<400> 28
Ala Ser Tyr Leu Ser Thr Ser Ser Ser Leu Asp Lys
1               5                   10

<210> 29
<211> 12
<212> PRT
<213> Artificial Sequence

<220>
<223> VH1D8 heavy chain variable region CDR3

<400> 29
Ala Ser Tyr Leu Ser Thr Ser Ser Ser Leu Asp Tyr
1               5                   10

<210> 30
<211> 12
<212> PRT
<213> Artificial Sequence

<220>
<223> VH1A11 heavy chain variable region CDR3

<400> 30
Ala Ser Tyr Leu Ser Thr Ser Ser Ser Leu Asp Asp
1               5                   10

<210> 31
<211> 12
<212> PRT
<213> Artificial Sequence

<220>
<223> VH1B12 heavy chain variable region CDR3

<400> 31
Ala Ser Tyr Leu Ser Thr Ser Phe Ser Leu Asp Tyr
1               5                   10

<210> 32
<211> 12
<212> PRT
<213> Artificial Sequence

<220>
<223> VH1E4 heavy chain variable region CDR3

<400> 32
Ala Ser Tyr Leu Ser Thr Ser Ser Ser Leu His Tyr
1               5                   10


<210> 33
<211> 12
<212> PRT
<213> Artificial Sequence

<220>
<223> VH1F6 heavy chain variable region CDR3

<400> 33
Ala Ser Phe Leu Ser Thr Ser Ser Ser Leu Glu Tyr
1               5                   10


<210> 34
<211> 12
<212> PRT
<213> Artificial Sequence

<220>
<223> 3C-H2 heavy chain variable region CDR3

<400> 34
Ala Ser Tyr Leu Ser Thr Ala Ser Ser Leu Glu Tyr
1               5                   10


<210> 35
<211> 12
<212> PRT
<213> Artificial Sequence

<220>
<223> VH1-D2.N heavy chain variable region CDR3

<400> 35
Val Ser Tyr Leu Ser Thr Ala Ser Ser Leu Asp Asn
1               5                   10


<210> 36
<211> 321
<212> DNA
<213> Artificial Sequence

<220>
<223> D2E7 light chain variable region

<400> 36
gacatccaga tgacccagtc tccatcctcc ctgtctgcat ctgtagggga cagagtcacc 60
atcacttgtc gggcaagtca gggcatcaga aattacttag cctggtatca gcaaaaacca 120
gggaaagccc ctaagctcct gatctatgct gcatccactt tgcaatcagg ggtcccatct 180
cggttcagtg gcagtggatc tgggacagat ttcactctca ccatcagcag cctacagcct 240
gaagatgttg caacttatta ctgtcaaagg tataaccgtg caccgtatac ttttggccag 300
gggaccaagg tggaaatcaa a                                           321

<210> 37

<211> 363
<212> DNA
<213> Artificial Sequence

<220>
<223> D2E7 heavy chain variable region

<400> 37
gaggtgcagc tggtggagtc tgggggaggc ttggtacagc ccggcaggtc cctgagactc 60
tcctgtgcgg cctctggatt cacctttgat gattatgcca tgcactgggt ccggcaagct 120
ccagggaagg gcctggaatg ggtctcagct atcacttgga atagtggtca catagactat 180
gcggactctg tggagggccg attcaccatc tccagagaca acgccaagaa ctccctgtat 240
ctgcaaatga acagtctgag agctgaggat acggccgtat attactgtgc gaaagtctcg 300
taccttagca ccgcgtcctc ccttgactat tggggccaag gtaccctggt caccgtctcg 360
agt 363

## Description

### Background of the Invention

Tumor necrosis factor α (TNFα) is a cytokine produced by numerous cell types, including monocytes and macrophages, that was originally identified based on its capacity to induce the necrosis of certain mouse tumors (see *e.g.,* Old, L. (1985) Science 230:630-632). Subsequently, a factor termed cachectin, associated with cachexia, was shown to be the same molecule as TNFα. TNFα has been implicated in mediating shock (see *e.g.,* Beutler, B. and Cerami, A. (1988) Annu. Rev. Biochem. 57:505-518; Beutler, B. and Cerami, A. (1989) Annu. Rev. Immunol. 7:625-655). Furthermore, TNFα has been implicated in the pathophysiology of a variety of other human diseases and disorders, including sepsis, infections, autoimmune diseases, transplant rejection and graft-versus-host disease (see *e.g.,* Vasilli, P. (1992) Annu. Rev. Immunol. 10:411-452; Tracey, K.J. and Cerami, A. (1994) Annu. Rev. Med. 45:491-503).

Because of the harmful role of human TNFα (hTNFα) in a variety of human disorders, therapeutic strategies have been designed to inhibit or counteract hTNFα activity. In particular, antibodies that bind to, and neutralize, hTNFα have been sought as a means to inhibit hTNFα activity. Some of the earliest of such antibodies were mouse monoclonal antibodies (mAbs), secreted by hybridomas prepared from lymphocytes of mice immunized with hTNFα (see *e.g.,* Hahn T; et al., (1985) Proc Natl Acad Sci USA 82: 3814-3818; Liang, C-M., et al. (1986) Biochem. Biophys. Res. Commun. 137:847-854; Hirai, M., et al. (1987) J. Immunol. Methods 96:57-62; Fendly, B.M., et al. (1987) Hybridoma 6:359-370; Möller, A., et al. (1990) Cytokine 2:162-169; U.S. Patent No. 5,231,024 to Moeller et al.; European Patent Publication No. 186 833 B1 by Wallach, D.; European Patent Application Publication No. 218 868 A1 by Old et al.; European Patent Publication No, 260 610 B1 by Moeller, A., et al.). While these mouse anti-hTNFα antibodies often displayed high affinity for hTNFα (*e.g.*, Kd ≤ 10⁻⁹M) and were able to neutralize hTNFα activity, their use *in vivo* may be limited by problems associated with administration of mouse antibodies to humans, such as short serum half life, an inability to trigger certain human effector functions and elicitation of an unwanted immune response against the mouse antibody in a human (the "human anti-mouse antibody" (HAMA) reaction).

In an attempt to overcome the problems associated with use of fully-murine antibodies in humans, murine anti-hTNFα antibodies have been genetically engineered to be more "human-like." For example, chimeric antibodies, in which the variable regions of the antibody chains are murine-derived and the constant regions of the antibody chains are human-derived, have been prepared (Knight, D.M, et al. (1993) Mol. Immunol. 30:1443-1453; PCT Publication No. WO 92/16553 by Daddona, P.E., et al.). Additionally, humanized antibodies, in which the hypervariable domains of the antibody variable regions are murine-derived but the remainder of the variable regions and the antibody constant regions are human-derived, have also been prepared (PCT Publication No. WO 92/11383 by Adair, J.R., et al.)*.* However, because these chimeric and humanized antibodies still retain some murine sequences, they still may elicit an unwanted immune reaction, the human anti-chimeric antibody (HACA) reaction, especially when administered for prolonged periods, *e.g.,* for chronic indications, such as rheumatoid arthritis (see *e.g.,* Elliott, M.J., et al. (1994) Lancet 344:1125-1127; Elliot, M.J., et al. (1994) Lancet 344,1105-1110).

A preferred hTNFα inhibitory agent to murine mAbs or derivatives thereof (*e.g*., chimeric or humanized antibodies) would be an entirely human anti-hTNFα antibody, since such an agent should not elicit the HAMA reaction, even if used for prolonged periods. Human monoclonal autoantibodies against hTNFα have been prepared using human hybridoma techniques (Boyle, P., et al. (1993) Cell. Immunol. 152:556-568; Boyle, P., et al. (1993) Cell. Immunol. 152:569-581; European Patent Application Publication No. 614 984 A2 by Boyle, et al.). However, these hybridoma-derived monoclonal autoantibodies were reported to have an affinity for hTNFα that was too low to calculate by conventional methods, were unable to bind soluble hTNFα and were unable to neutralize hTNFα-induced cytotoxicity (see Boyle, *et al.; supra*)*.* Moreover, the success of the human hybridoma technique depends upon the natural presence in human peripheral blood of lymphocytes producing autoantibodies specific for hTNFα. Certain studies have detected serum autoantibodies against hTNFα in human subjects (Fomsgaard, A., et al. (1989) Scand. J. Immunol. 30:219-223; Bendtzen, K., et al. (1990) Prog. Leukocyte Biol. 10B:447-452), whereas others have not (Leusch, H-G., et al. (1991) J. Immunol. Methods 139:145-147).

Alternative to naturally-occurring human anti-hTNFα antibodies would be a recombinant hTNFα antibody. Recombinant human antibodies that bind hTNFα with relatively low affinity (*i.e.*, K_{d} ∼10⁻⁷M) and a fast off rate (*i.e.*, T_{off}∼ 10⁻² sec⁻¹) have been described (Griffiths, A.D., et al. (1993) EMBO J. 12:725-734). However, because of their relatively fast dissociation kinetics, these antibodies may not be suitable for therapeutic use. Additionally, a recombinant human anti-hTNFα has been described that does not neutralize hTNFα activity, but rather enhances binding of hTNFα to the surface of cells and enhances internalization of hTNFα (Lidbury, A., et al. (1994) Biotechnol. Ther. 5:27-45; PCT Publication No. WO 92/03145 by Aston, R. et al.)

Recombinant human antibodies that bind soluble hTNFα with high affinity and slow dissociation kinetics and that have the capacity to neutralize hTNFα activity, including hTNFα-induced cytotoxicity (*in vitro* and *in vivo*) and hTNFα-induced cell activation, have also been described (see U.S. Patent No. 6,090,382). Typical protocols for administering antibodies are performed intravenously on a weekly basis. Weekly dosing with antibodies and/or any drug can be costly, cumbersome, and result in an increase in the number of side effects due to the frequency of administration. Intravenous administration also has limitations in that the administration is usually provided by someone with medical training.

### Summary of the Invention

The present invention provides methods for administering an anti-TNFα antibody with one or more drug for treating TNFα associated disorders, for example, sepsis, an autoimmune disease (*e.g.*, rheumatoid arthritis, allergy, multiple sclerosis, autoimmune diabetes, autoimmune uveitis and nephrotic syndrome), an infectious disease, a malignancy, transplant rejection or graft-versus-host disease, a pulmonary disorder, a bone disorder, an intestinal disorder, a disorder of the central nervous system, a cardiac disorder. Preferably the autoimmune disease that is treated is rheumatoid arthritis.

The antibody may be administered before, with or after the administration of the other drug(s), A method of this invention comprises any order of administration of the antibody and other drug(s) and by any means of administration. Preferably the antibody is administered in biweekly dosing regimens for the treatment of TNFα associated disorders, preferably via subcutaneous route.

Preferably the other drug is a Disease Modifying Anti-Rheumatic Drug (DMARD) or a Nonsteroidal Antiinflammatory Drug (NSAID) or a steroid or any combination thereof. Preferred examples of a DMARD are hydroxychloroquine, leflunomide, methotrexate, parenteral gold, oral gold and sulfasalazine. These methods include utilizing a combination therapy wherein human antibodies are administered to a subject with another therapeutic agent, such as one or more additional' antibodies that bind other targets (*e.g.*, antibodies that bind other cytokines or that bind cell surface molecules), one or more cytokines, soluble TNFα receptor (see *e.g.,* PCT Publication No. WO 94/06476), etanercept (ENBREL^{™}) from Immunex Corporation and infliximab (REMICADE^{™}) from Centocor, Inc., and/or one or more chemical agents that inhibit hTNFα production or activity (such as cyclohexane-ylidene derivatives as described in PCT Publication No. WO 93/19751).

Another aspect of the invention pertains to a pharmaceutical composition comprising an anti-TNFα antibody and one or more drugs useful for treating an autoimmune disorder and a pharmaceutically acceptable carrier.

Another aspect of the invention pertains to kits containing a pharmaceutical composition comprising an anti-TNFα antibody and a pharmaceutically acceptable carrier and one or more pharmaceutical compositions each comprising a drug useful for treating an autoimmune disorder and a pharmaceutically acceptable carrier. Alternatively, the kit comprises a single pharmaceutical composition comprising an anti-TNFα antibody, one or more drugs useful for treating an autoimmune disorder and a pharmaceutically acceptable carrier. The kits contain instructions for dosing of the pharmaceutical compositions for the treatment of a disorder in which the administration of an anti-TNFα antibody is beneficial, such as an autoimmune disorder, especially rheumatoid arthritis.

The antibodies or an antigen-binding portion thereof preferably are recombinant human antibodies that specifically bind to human TNFα. The antibodies are preferably recombinant human antibodies that specifically bind to human TNFα. The antibodies of the invention are characterized by binding to hTNFα with high affinity and slow dissociation kinetics and by neutralizing hTNFα activity, including hTNFα-induced cytotoxicity (*in vitro* and *in vivo*) and hTNFα-induced cellular activation. The antibody or antigen-binding portion thereof preferably dissociates from human TNFα with a K_{d} of 1 x 10⁻⁸ M or less and a K_{off} rate constant of 1 x 10⁻³ s⁻¹ or less, both determined by surface plasmon resonance, and neutralizes human TNFα cytotoxicity in a standard *in vitro* L929 assay with an IC₅₀ of 1 x 10⁻⁷ M or less. More preferably, the isolated human antibody, or antigen-binding portion thereof, dissociates from human TNFα with a K_{off} of 5 x 10⁻⁴ s⁻¹ or less, or even more preferably, with a K_{off} of 1 x 10⁻⁴ s⁻¹ or less. More preferably, the isolated human antibody, or antigen-binding portion thereof, neutralizes human TNFα cytotoxicity in a standard *in vitro* L929 assay with an IC₅₀ of 1 x 10⁻⁸ M or less, even more preferably with an IC₅₀ of 1 x 10⁻⁹ M or less and still more preferably with an IC₅₀ of 1 x 10⁻¹⁰ M or less. The antibodies can be full-length (*e.g.*, an IgG1 or IgG4 antibody) or can comprise only an antigen-binding portion (*e.g.*, a Fab, F(ab')₂, scFv fragment or single domain). The most preferred recombinant antibody of the invention, termed D2E7, has a light chain CDR3 domain comprising the amino acid sequence of SEQ ID NO: 3 and a heavy chain CDR3 domain comprising the amino acid sequence of SEQ ID NO: 4. Preferably, the D2E7 antibody has a light chain variable region (LCVR) comprising the amino acid sequence of SEQ ID NO: 1 and a heavy chain variable region (HCVR) comprising the amino acid sequence of SEQ ID NO: 2. These antibodies are described in U.S. Patent No. 6,090,382, incorporated in its entirety herein by reference.

Preferably, the antibody or antigen-binding portion thereof has the following characteristics:
a) dissociates from human TNFα with a K_{off} of 1 x 10⁻³ s⁻¹ or less, as determined by surface plasmon resonance;
b) has a light chain CDR3 domain comprising the amino acid sequence of SEQ ID NO: 3, or modified from SEQ ID NO; 3 by a single alanine substitution at position 1, 4, 5, 7 or 8 or by one to five conservative amino acid substitutions at positions 1, 3, 4, 6, 7, 8 and/or 9;
c) has a heavy chain CDR3 domain comprising the amino acid sequence of SEQ ID NO: 4, or modified from SEQ ID NO: 4 by a single alanine substitution at position 2, 3, 4, 5, 6, 8, 9, 10 or 11 or by one to five conservative amino acid substitutions at positions 2, 3, 4, 5, 6, 8, 9, 10, 11 and/or 12.

More preferably, the antibody, or antigen-binding portion thereof, dissociates from human TNFα with a K_{off} of 5 x 10⁻⁴ s⁻¹ or less. Still more preferably, the antibody, or antigen-binding portion thereof, dissociates from human TNFα with a K_{off} of 1 x 10⁻⁴ s⁻¹ or less.

The antibody or antigen-binding portion thereof preferably contains an LCVR having CDR3 domain comprising the amino acid sequence of SEQ ID NO: 3, or modified from SEQ ID NO: 3 by a single alanine substitution at position 1,4,5,7 or 8, and with an HCVR having a CDR3 domain comprising the amino acid sequence of SEQ ID NO: 4, or modified from SEQ ID NO: 4 by a single alanine substitution at position 2, 3, 4, 5, 6, 8, 9, 10 or 11. More preferably, the LCVR further has a CDR2 domain comprising the amino acid sequence of SEQ ID NO: 5 and the HCVR further has a CDR2 domain comprising the amino acid sequence of SEQ ID NO: 6. Still more preferably, the LCVR further has CDR1 domain comprising the amino acid sequence of SEQ ID NO: 7 and the HCVR has a CDR1 domain comprising the amino acid sequence of SEQ ID NO: 8.

The antibody or antigen-binding portion thereof preferably contains an LCVR comprising the amino acid sequence of SEQ ID NO: 1 and an HCVR comprising the amino acid sequence of SEQ ID NO: 2. In certain embodiments, the antibody has an IgG1 heavy chain constant region or an IgG4 heavy chain constant region. In yet other embodiments, the antibody is a Fab fragment, an F(ab')₂ fragment or a single chain Fv fragment.

In still other embodiments, the invention provides methods of treating disorders in which the administration of an anti-TNFα antibody is beneficial by subcutaneously administering to the subject, biweekly, one or more anti-TNFα antibodies, or antigen-binding portions thereof. The antibody or antigen-binding portion thereof preferably contains an LCVR having CDR3 domain comprising an amino acid sequence selected from the group consisting of SEQ ID NO: 3, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26 or with an HCVR having a CDR3 domain comprising an amino acid sequence selected from the group consisting of SEQ ID NO: 4, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34 and SEQ ID NO: 35.

Most preferred of the foregoing embodiments of this invention is where the antibody is D2E7 administered biweekly subcutaneously at a dose of 40 mg. The dose of the one or more of the other drug is in accordance with its effective dose.

### Detailed Description of the Invention

This invention pertains to methods of treating disorders in which the administration of an anti-TNFα antibody is beneficial comprising the administration of isolated human antibodies, or antigen-binding portions thereof, that bind to human TNFα with high affinity, a low off rate and high neutralizing capacity such that the disorder is treated, with one or more other drugs.

In order that the present invention may be more readily understood, certain terms are first defined.

The term "dosing", as used herein, refers to the administration of a substance (*e.g.,* an anti-TNFα antibody) to achieve a therapeutic objective (*e.g.*, the treatment of a TNFα-associated disorder).

The terms "biweekly dosing regimen", "biweekly dosing", and "biweekly administration", as used herein, refer to the time course of administering a substance (*e.g.,* an anti-TNFα antibody) to a subject to achieve a therapeutic objective (*e*.*g*., the treatment of a TNFα-associated disorder). The biweekly dosing regimen is not intended to include a weekly dosing regimen. Preferably, the substance is administered every 9-19 days, more preferably, every 11-17 days, even more preferably, every 13-15 days, and most preferably, every 14 days.

The term "combination therapy", as used herein, refers to the administration of two or more therapeutic substances, *e.g.,* an anti-TNFα antibody and another drug, such as a DMARD or NSAID, The other drug(s) may be administered concomitant with, prior to, or following the administration of an anti-TNFα antibody.

The term "human TNFα" (abbreviated herein as hTNFα, or simply hTNF), as used herein, is intended to refer to a human cytokine that exists as a 17 kD secreted form and a 26 kD membrane associated form, the biologically active form of which is composed of a trimer of noncovalently bound 17 kD molecules. The structure of TNFα is described further in, for example, Pennica, D., et al. (1984) Nature 312:724-729; Davis, J.M., et al. (1987) Biochemistry 26:1322-1326; and Jones, E.Y., et al. (1989) Nature 338:225-228. The term human TNFα is intended to include recombinant human TNFα (rhTNFα), which can be prepared by standard recombinant expression methods or purchased commercially (R & D Systems, Catalog No. 210-TA, Minneapolis, MN).

The term "antibody", as used herein, is intended to refer to immunoglobulin molecules comprised of four polypeptide chains, two heavy (H) chains and two light (L) chains inter-connected by disulfide bonds. Each heavy chain is comprised of a heavy chain variable region (abbreviated herein as HCVR or VH) and a heavy chain constant region. The heavy chain constant region is comprised of three domains, CH1, CH2 and CH3. Each light chain is comprised of a light chain variable region (abbreviated herein as LCVR or VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4.

The term "antigen-binding portion" of an antibody (or simply "antibody portion"), as used herein, refers to one or more fragments of an antibody that retain the ability to specifically bind to an antigen (*e.g.*, hTNFα). It has been shown that the antigen-binding function of an antibody can be performed by fragments of a full-length antibody. Examples of binding fragments encompassed within the term "antigen-binding portion" of an antibody include (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546 ), which consists of a VH domain; and (vi) an isolated complementarity determining region (CDR), Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see *e.g.,* Bird et al. (1988) Science 242:423-426; and Huston et al. (1988) Proc. Natl. Acad. Sci. USA 85:5879-5883). Such single chain antibodies are also intended to be encompassed within the term "antigen-binding portion" of an antibody. Other forms of single chain antibodies, such as diabodies are also encompassed. Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (see *e.g.,* Holliger, P., et al. (1993) Proc. Natl. Acad. Sci. USA 90:6444-6448; Poljak, R.J., et al. (1994) Structure 2:1121-1123).

Still further, an antibody or antigen-binding portion thereof may be part of a larger immunoadhesion molecules, formed by covalent or noncovalent association of the antibody or antibody portion with one or more other proteins or peptides, Examples of such immunoadhesion molecules include use of the streptavidin core region to make a tetrameric scFv molecule (Kipriyanov, S.M., et al. (1995) Human Antibodies and Hybridomas 6:93-101) and use of a cysteine residue, a marker peptide and a C-terminal polyhistidine tag to make bivalent and biotinylated scFv molecules (Kipriyanov, S.M., et al, (1994) Mol. Immunol. 31:1047-1058). Antibody portions, such as Fab and F(ab')₂ fragments, can be prepared from whole antibodies using conventional techniques, such as papain or pepsin digestion, respectively, of whole antibodies. Moreover, antibodies, antibody portions and immunoadhesion molecules can be obtained using standard recombinant DNA techniques, as described herein.

The term "human antibody", as used herein, is intended to include antibodies having variable and constant regions derived from human germline immunoglobulin sequences. The human antibodies of the invention may include amino acid residues not encoded by human germline immunoglobulin sequences (*e.g.*, mutations introduced by random or site-specific mutagenesis *in vitro* or by somatic mutation *in vivo*), for example in the CDRs and in particular CDR3. However, the term "human antibody", as used herein, is not intended to include antibodies in which CDR sequences derived from the germline of another mammalian species, such as a mouse, have been grafted onto human framework sequences.

The term "recombinant human antibody", as used herein, is intended to include all human antibodies that are prepared, expressed, created or isolated by recombinant means, such as antibodies expressed using a recombinant expression vector transfected into a host cell (described further in Section II, below), antibodies isolated from a recombinant, combinatorial human antibody library (described further in Section III, below), antibodies isolated from an animal (*e.g.,* a mouse) that is transgenic for human immunoglobulin genes (see *e.g.,* Taylor, L.D., et al. (1992) Nucl. Acids Res. 20:6287-6295) or antibodies prepared, expressed, created or isolated by any other means that involves splicing of human immunoglobulin gene sequences to other DNA sequences. Such recombinant human antibodies have variable and constant regions derived from human germline immunoglobulin sequences. In certain embodiments, however, such recombinant human antibodies are subjected to *in vitro* mutagenesis (or, when an animal transgenic for human Ig sequences is used, *in vivo* somatic mutagenesis) and thus the amino acid sequences of the VH and VL regions of the recombinant antibodies are sequences that, while derived from and related to human germline VH and VL sequences, may not naturally exist within the human antibody germline repertoire *in vivo*.

An "isolated antibody", as used herein, is intended to refer to an antibody that is substantially free of other antibodies having different antigenic specificities (*e.g.*, an isolated antibody that specifically binds hTNFα is substantially free of antibodies that specifically_bind antigens other than hTNFα). An isolated antibody that specifically binds hTNFα may, however, have cross-reactivity to other antigens, such as hTNFα molecules from other species (discussed in further detail below). Moreover, an isolated antibody may be substantially free of other cellular material and/or chemicals.

A "neutralizing antibody", as used herein (or an "antibody that neutralized hTNFα activity"), is intended to refer to an antibody whose binding to hTNFα results in inhibition of the biological activity of hTNFα. This inhibition of the biological activity of hTNFα can be assessed by measuring one or more indicators of hTNFα biological activity, such as hTNFα-induced cytotoxicity (either *in vitro* or *in vivo*), hTNFα-induced cellular activation and hTNFα binding to hTNFα receptors. These indicators of hTNFα biological activity can be assessed by one or more of several standard *in vitro* or *in vivo* assays known in the art (see U.S. Patent No. 6,090,382). Preferably, the ability of an antibody to neutralize hTNFα activity is assessed by inhibition of hTNFα-induced cytotoxicity of L929 cells. As an additional or alternative parameter of hTNFα activity, the ability of an antibody to inhibit hTNFα-induced expression of ELAM-1 on HUVEC, as a measure of hTNFα-induced cellular activation, can be assessed.

The term "surface plasmon resonance", as used herein, refers to an optical phenomenon that allows for the analysis of real-time biospecific interactions by detection of alterations in protein concentrations within a biosensor matrix, for example using the BIAcore system (Pharmacia Biosensor AB, Uppsala, Sweden and Piscataway, NJ). For further descriptions, see Example 1 and Jönsson, U., et al. (1993) Ann. Biol. Clin. 51:19-26; Jönsson, U., et al. (1991) Biotechniques 11:620-627; Johnsson, B., et al. (1995) J. Mol, Recognit. 8:125-131; and Johnnson, B., et al. (1991) Anal. Biochem. 198:268-277.

The term "K_{off}", as used herein, is intended to refer to the off rate constant for dissociation of an antibody from the antibody/antigen complex.

The term "K_{d}", as used herein, is intended to refer to the dissociation constant of a particular antibody-antigen interaction.

The term "nucleic acid molecule", as used herein, is intended to include DNA molecules and RNA molecules. A nucleic acid molecule may be single-stranded or double-stranded, but preferably is double-stranded DNA.

The term "isolated nucleic acid molecule", as used herein in reference to nucleic acids encoding antibodies or antibody portions (*e.g*., VH, VL, CDR3) that bind hTNFα, is intended to refer to a nucleic acid molecule in which the nucleotide sequences encoding the antibody or antibody portion are free of other nucleotide sequences encoding antibodies or antibody portions that bind antigens other than hTNFα, which other sequences may naturally flank the nucleic acid in human genomic DNA. Thus, for example, an isolated nucleic acid of the invention encoding a VH region of an anti-hTNFα antibody contains no other sequences encoding other VH regions that bind antigens other than hTNFα.

The term "vector", as used herein, is intended to refer to a nucleic acid molecule capable of transporting another nucleic acid to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (*e.g.*, bacterial vectors having a bacterial origin of replication and episomal mammalian vectors). Other vectors (*e.g.*, non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "expression vectors"). In general, expression vectors of utility in recombinant DNA techniques are often in the form of plasmids. In the present specification, "plasmid" and "vector" may be used interchangeably as the plasmid is the most commonly used form of vector. However, the invention is intended to include such other forms of expression vectors, such as viral vectors (e.g., replication defective retroviruses, adenoviruses and adeno-associated viruses), which serve equivalent functions.

The term "recombinant host cell" (or simply "host cell"), as used herein, is intended to refer to a cell into which a recombinant expression vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein.

Various aspects of the invention are described in further detail herein.

This invention provides methods of treating disorders in which the administration of an anti-TNFα antibody is beneficial. These methods include the biweekly, subcutaneous administration of isolated human antibodies, or antigen-binding portions thereof, that bind to human TNFα with high affinity, a low off rate and high neutralizing capacity along with one or more other drugs that are useful in treating an autoimmune disease, especially rheumatoid arthritis. Preferably, the human antibodies of the invention are recombinant, neutralizing human anti-hTNFα antibodies. The most preferred recombinant, neutralizing antibody of the invention is referred to herein as D2E7 (the amino acid sequence of the D2E7 VL region is shown in SEQ ID NO:1; the amino acid sequence of the D2E7 VH region is shown in SEQ ID NO: 2), which is administered at a dose of 40 mg. The properties of D2E7 have been described in Salfeld et al., U.S. patent No. 6,090,382, which is incorporated by reference herein.

In one aspect, the invention pertains to treating disorders in which the administration of an anti-TNFα antibody and one or more other drugs that are useful in treating an autoimmune disorder is beneficial. These treatments include the biweekly, subcutaneous administration of D2E7 antibodies and antibody portions thereof, D2E7-related antibodies and antibody portions thereof, and other human antibodies and antibody portions thereof with equivalent properties to D2E7, such as high affinity binding to hTNFα with low dissociation kinetics and high neutralizing capacity. In one embodiment, the invention provides treatment with an isolated human antibody, or an antigen-binding portion thereof, that dissociates from human TNFα with a K_{d} of 1 x 10⁻⁸ M or less and a K_{off} rate constant of 1 x 10⁻³ s⁻¹ or less, both determined by surface plasmon resonance, and neutralizes human TNFα cytotoxicity in a standard *in vitro* L929 assay with an IC₅₀ of 1 x 10⁻⁷ M or less, More preferably, the isolated human antibody, or antigen-binding portion thereof, dissociates from human TNFα with a K_{off} of 5 x 10⁻⁴ s⁻¹ or less, or even more preferably, with a K_{off} of 1 x 10⁻⁴ s⁻¹ or less. More preferably, the isolated human antibody, or antigen-binding portion thereof, neutralizes human TNFα cytotoxicity in a standard *in vitro* L929 assay with an IC₅₀ of 1 x 10⁻⁸ M or less, even more preferably with an IC₅₀ of 1 x 10⁻⁹ M or less and still more preferably with an IC₅₀ of 1 x 10⁻¹⁰ M or less. In a preferred embodiment, the antibody is an isolated human recombinant antibody, or an antigen-binding portion thereof.

It is well known in the art that antibody heavy and light chain CDR3 domains play an important role in the binding specificity/affinity of an antibody for an antigen. Accordingly, in another aspect, the invention pertains to methods of treating disorders in which the administration of an anti-TNFα antibody is beneficial by subcutaneous administration of human antibodies that have slow dissociation kinetics for association with hTNFα and that have light and heavy chain CDR3 domains that structurally are identical to or related to those of D2E7. Position 9 of the D2E7 VL CDR3 can be occupied by Ala or Thr without substantially affecting the K_{off}. Accordingly, a consensus motif for the D2E7 VL CDR3 comprises the amino acid sequence: Q-R-Y-N-R-A-P-Y-(T/A) (SEQ ID NO: 3). Additionally, position 12 of the D2E7 VH CDR3 can be occupied by Tyr or Asn, without substantially affecting the K_{off}. Accordingly, a consensus motif for the D2E7 VH CDR3 comprises the amino acid sequence: V-S-Y-L-S-T-A-S-S-L-D-(Y/N) (SEQ ID NO: 4). Moreover, as demonstrated in Example 2, the CDR3 domain of the D2E7 heavy and light chains is amenable to substitution with a single alanine residue (at position 1, 4, 5, 7 or 8 within the VL CDR3 or at position 2, 3, 4, 5, 6, 8, 9, 10 or 11 within the VH CDR3) without substantially affecting the K_{off}. Still further, the skilled artisan will appreciate that, given the amenability of the D2E7 VL and VH CDR3 domains to substitutions by alanine, substitution of other amino acids within the CDR3 domains may be possible while still retaining the low off rate constant of the antibody, in particular substitutions with conservative amino acids. A "conservative amino acid substitution", as used herein, is one in which one amino acid residue is replaced with another amino acid residue having a similar side chain. Families of amino acid residues having similar side chains have been defined in the art, including basic side chains (*e.g.,* lysine, arginine, histidine), acidic side chains (*e.g*., aspartic acid, glutamic acid), uncharged polar side chains (*e.g.*, glycine, asparagine, glutamine, serine, threonine, tyrosine, cysteine), nonpolar side chains (*e.g.,* alanine, valine, leucine, isoleucine, proline, phenylalanine, methionine, tryptophan), beta-branched side chains (*e.g*., threonine, valine, isoleucine) and aromatic side chains (*e*.*g*., tyrosine, phenylalanine, tryptophan, histidine). Preferably, no more than one to five conservative amino acid substitutions are made within the D2E7 VL and/or VH CDR3 domains. More preferably, no more than one to three conservative amino acid substitutions are made within the D2E7 VL and/or VH CDR3 domains. Additionally, conservative amino acid substitutions should not be made at amino acid positions critical for binding to hTNFα. Positions 2 and 5 of the D2E7 VL CDR3 and positions 1 and 7 of the D2E7 VH CDR3 appear to be critical for interaction with hTNFα and thus, conservative amino acid substitutions preferably are not made at these positions (although an alanine substitution at position 5 of the D2E7 VL CDR3 is acceptable, as described above) (*see* U.S. Patent No. 6,090,382).

Accordingly, in another embodiment, the invention provides methods of treating disorders in which the administration of an anti-TNFα antibody with one or more other drugs that are useful in treating an autoimmune disease is beneficial by the biweekly, subcutaneous administration of an isolated human antibody, or antigen-binding portion thereof. The antibody or antigen-binding portion thereof preferably contains the following characteristics:
a) dissociates from human TNFα with a K_{off} rate constant of 1 x 10⁻³ s⁻¹ or less, as determined by surface plasmon resonance;
b) has a light chain CDR3 domain comprising the amino acid sequence of SEQ ID NO: 3, or modified from SEQ ID NO: 3 by a single alanine substitution at position 1, 4,5,7 or 8 or by one to five conservative amino acid substitutions at positions 1,3,4,6, 7, 8 and/or 9;
c) has a heavy chain CDR3 domain comprising the amino acid sequence of SEQ ID NO: 4, or modified from SEQ ID NO: 4 by a single alanine substitution at position 2, 3, 4, 5, 6, 8, 9, 10 or 11 or by one to five conservative amino acid substitutions at positions 2, 3, 4, 5, 6, 8, 9, 10, 11 and/or 12.

More preferably, the antibody, or antigen-binding portion thereof, dissociates from human TNFα with a K_{off} of 5 x 10⁻⁴ s⁻¹ or less. Even more preferably, the antibody, or antigen-binding portion thereof, dissociates from human TNFα with a K_{off} of 1 x 10⁻⁴ s⁻¹ or less.

In yet another embodiment, the antibody or antigen-binding portion thereof preferably contains a light chain variable region (LCVR) having a CDR3 domain comprising the amino acid sequence of SEQ ID NO: 3, or modified from SEQ ID NO: 3 by a single alanine substitution at position 1, 4, 5, 7 or 8, and with a heavy chain variable region (HCVR) having a CDR3 domain comprising the amino acid sequence of SEQ ID NO: 4, or modified from SEQ ID NO: 4 by a single alanine substitution at position 2, 3, 4, 5, 6, 8, 9, 10 or 11. Preferably, the LCVR further has a CDR2 domain comprising the amino acid sequence of SEQ ID NO: 5 (*i.e.*, the D2E7 VL CDR2) and the HCVR further has a CDR2 domain comprising the amino acid sequence of SEQ ID NO: 6 (*i.e.,* the D2E7 VH CDR2). Even more preferably, the LCVR further has CDR1 domain comprising the amino acid sequence of SEQ ID NO: 7 (*i.e.*, the D2E7 VL CDR1) and the HCVR has a CDR1 domain comprising the amino acid sequence of SEQ ID NO: 8 (*i.e.*, the D2E7 VH CDR1). The framework regions for VL preferably are from the V_{κ}I human germline family, more preferably from the A20 human germline Vk gene and most preferably from the D2E7 VL framework sequences shown in Figures 1A and 1B of U.S. Patent No. 6,090,382. The framework regions for VH preferably are from the V_{H}3 human germline family, more preferably from the DP-31 human germline VH gene and most preferably from the D2E7 VH framework sequences shown in Figures 2A and 2B of U.S. Patent No. 6,090,382.

In still another embodiment, the antibody or antigen-binding portion thereof preferably contains a light chain variable region (LCVR) comprising the amino acid sequence of SEQ ID NO: 1 (*i*.*e.*, the D2E7 VL) and a heavy chain variable region (HCVR) comprising the amino acid sequence of SEQ ID NO: 2 (*i.e.,* the D2E7 VH). In certain embodiments, the antibody comprises a heavy chain constant region, such as an IgG1, IgG2, IgG3, IgG4, IgA, IgE, IgM or IgD constant region. Preferably, the heavy chain constant region is an IgG1 heavy chain constant region or an IgG4 heavy chain constant region. Furthermore, the antibody can comprise a light chain constant region, either a kappa light chain constant region or a lambda light chain constant region. Preferably, the antibody comprises a kappa light chain constant region. Alternatively, the antibody portion can be, for example, a Fab fragment or a single chain Fv fragment.

In still other embodiments, the antibody or antigen-binding portion thereof preferably contains D2E7-related VL and VH CDR3 domains, for example, antibodies, or antigen-binding portions thereof, with a light chain variable region (LCVR) having a CDR3 domain comprising an amino acid sequence selected from the group consisting of SEQ ID NO: 3, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25 and SEQ ID NO: 26 or with a heavy chain variable region (HCVR) having a CDR3 domain comprising an amino acid sequence selected from the group consisting of SEQ ID NO: 4, SEQ ID NO; 27, SEQ ID NO: 28, SEQ ID NO: 29, SEQ ID NO: 30, SEQ ID NO: 31, SEQ ID NO: 32, SEQ ID NO: 33, SEQ ID NO: 34 and SEQ ID NO: 35.

An antibody or antibody portion of the invention can be derivatized or linked to another functional molecule (*e.g.,* another peptide or protein). Accordingly, the antibodies and antibody portions of the invention are intended to include derivatized and otherwise modified forms of the human anti-hTNFα antibodies described herein, including immunoadhesion molecules. For example, an antibody or antibody portion of the invention can be functionally linked (by chemical coupling, genetic fusion, noncovalent association or otherwise) to one or more other molecular entities, such as another antibody (*e.g.*, a bispecific antibody or a diabody), a detectable agent, a cytotoxic agent, a pharmaceutical agent, and/or a protein or peptide that can mediate associate of the antibody or antibody portion with another molecule (such as a streptavidin core region or a polyhistidine tag).

One type of derivatized antibody is produced by crosslinking two or more antibodies (of the same type or of different types, *e.g.,* to create bispecific antibodies). Suitable crosslinkers include those that are heterobifunctional, having two distinctly reactive groups separated by an appropriate spacer (*e.g.*, m-maleimidobenzoyl-N-hydroxysuccinimide ester) or homobifunctional (*e.g*., disuccinimidyl suberate). Such linkers are available from Pierce Chemical Company, Rockford, IL.

Useful detectable agents with which an antibody or antibody portion of the invention may be derivatized include fluorescent compounds. Exemplary fluorescent detectable agents include fluorescein, fluorescein isothiocyanate, rhodamine, 5-dimethylamine-1-napthalenesulfonyl chloride, phycoerythrin and the like. An antibody may also be derivatized with detectable enzymes, such as alkaline phosphatase, horseradish peroxidase, glucose oxidase and the like. When an antibody is derivatized with a detectable enzyme, it is detected by adding additional reagents that the enzyme uses to produce a detectable reaction product. For example, when the detectable agent horseradish peroxidase is present, the addition of hydrogen peroxide and diaminobenzidine leads to a colored reaction product, which is detectable. An antibody may also be derivatized with biotin, and detected through indirect measurement of avidin or streptavidin binding.

An antibody, or antibody portion, of the invention can be prepared by recombinant expression of immunoglobulin light and heavy chain genes in a host cell. To express an antibody recombinantly, a host cell is transfected with one or more recombinant expression vectors carrying DNA fragments encoding the immunoglobulin light and heavy chains of the antibody such that the light and heavy chains are expressed in the host cell and, preferably, secreted into the medium in which the host cells are cultured, from which medium the antibodies can be recovered. Standard recombinant DNA methodologies are used to obtain antibody heavy and light chain genes, incorporate these genes into recombinant expression vectors and introduce the vectors into host cells, such as those described in Sambrook, Fritsch and Maniatis (eds), Molecular Cloning; A Laboratory Manual, Second Edition, Cold Spring Harbor, N.Y., (1989), Ausubel, F.M. et al. (eds.) Current Protocols in Molecular Biology, Greene Publishing Associates, (1989) and in U.S. Patent No. 4,816,397 by Boss et al.

To express D2E7 or a D2E7-related antibody, DNA fragments encoding the light and heavy chain variable regions are first obtained. These DNAs can be obtained by amplification and modification of germline light and heavy chain variable sequences using the polymerase chain reaction (PCR). Germline DNA sequences for human heavy and light chain variable region genes are known in the art (see *e.g.*, the "Vbase" human germline sequence database; see also Kabat, E.A., et al. (1991) Sequences of Proteins of Immunological Interest, Fifth Edition, U.S. Department of Health and Human Services, NIH Publication No. 91-3242; Tomlinson, I.M., et al. (1992) "The Repertoire of Human Germline VH Sequences Reveals about Fifty Groups of VH Segments with Different Hypervariable Loops" J. Mol. Biol. 227:776-798; and Cox, J.P.L. et al. (1994) "A Directory of Human Germ-line V78 Segments Reveals a Strong Bias in their Usage" Eur. J. Immunol. 24:827-836; the contents of each of which are expressly incorporated herein by reference). To obtain a DNA fragment encoding the heavy chain variable region of D2E7, or a D2E7-related antibody, a member of the V_{H}3 family of human germline VH genes is amplified by standard PCR. Most preferably, the DP-31 VH germline sequence is amplified. To obtain a DNA fragment encoding the light chain variable region of D2E7, or a D2E7-related antibody, a member of the V_{κ}I family of human germline VL genes is amplified by standard PCR. Most preferably, the A20 VL germline sequence is amplified. PCR primers suitable for use in amplifying the DP-31 germline VH and A20 germline VL sequences can be designed based on the nucleotide sequences disclosed in the references cited *supra,* using standard methods.

Once the germline VH and VL fragments are obtained, these sequences can be mutated to encode the D2E7 or D2E7-related amino acid sequences disclosed herein. The amino acid sequences encoded by the germline VH and VL DNA sequences are first compared to the D2E7 or D2E7-related VH and VL amino acid sequences to identify amino acid residues in the D2E7 or D2E7-related sequence that differ from germline. Then, the appropriate nucleotides of the germline DNA sequences are mutated such that the mutated germline sequence encodes the D2E7 or D2E7-related amino acid sequence, using the genetic code to determine which nucleotide changes should be made. Mutagenesis of the germline sequences is carried out by standard methods, such as PCR-mediated mutagenesis (in which the mutated nucleotides are incorporated into the PCR primers such that the PCR product contains the mutations) or site-directed mutagenesis.

Once DNA fragments encoding D2E7 or D2E7-related VH and VL segments are obtained (by amplification and mutagenesis of germline VH and VL genes, as described above), these DNA fragments can be further manipulated by standard recombinant DNA techniques, for example to convert the variable region genes to full-length antibody chain genes, to Fab fragment genes or to a scFv gene. In these manipulations, a VL- or VH-encoding DNA fragment is operatively linked to another DNA fragment encoding another protein, such as an antibody constant region or a flexible linker. The term "operatively linked", as used in this context, is intended to mean that the two DNA fragments are joined such that the amino acid sequences encoded by the two DNA fragments remain in-frame.

The isolated DNA encoding the VH region can be converted to a full-length heavy chain gene by operatively linking the VH-encoding DNA to another DNA molecule encoding heavy chain constant regions (CH1, CH2 and CH3). The sequences of human heavy chain constant region genes are known in the art (see *e*.*g*., Kabat, E.A., et al. (1991) Sequences of Proteins of Immunological Interest, Fifth Edition, U.S. Department of Health and Human Services, NIH Publication No. 91-3242) and DNA fragments encompassing these regions can be obtained by standard PCR amplification. The heavy chain constant region can be an Ido1, IgG2, IgG3, IgG4, IgA, IgE, IgM or IgD constant region, but most preferably is an IgG1 or IgG4 constant region. For a Fab fragment heavy chain gene, the VH-encoding DNA can be operatively linked to another DNA molecule encoding only the heavy chain CH1 constant region.

The isolated DNA encoding the VL region can be converted to a full-length light chain gene (as well as a Fab light chain gene) by operatively linking the VL-encoding DNA to another DNA molecule encoding the light chain constant region, CL. The sequences of human light chain constant region genes are known in the art (see *e*.*g*., Kabat, E.A., et al. (1991) Sequences of Proteins of Immunological Interest, Fifth Edition, U.S. Department of Health and Human Services, NIH Publication No. 91-3242) and DNA fragments encompassing these regions can be obtained by standard PCR amplification. The light chain constant region can be a kappa or lambda constant region, but most preferably is a kappa constant region.

To create a scFv gene, the VH- and VL-encoding DNA fragments are operatively linked to another fragment encoding a flexible linker, *e*.*g*., encoding the amino acid sequence (Gly₄-Ser)₃, such that the VH and VL sequences can be expressed as a contiguous single-chain protein, with the VL and VH regions joined by the flexible linker (see *e.g.,* Bird et al. (1988) Science 242:423-426; Huston et al. (1988) Proc. Natl. Acad. Sci. USA 85:5879-5883; McCafferty et al., Nature (1990) 348:552-554).

To express the antibodies, or antibody portions of the invention, DNAs encoding partial or full-length light and heavy chains, obtained as described above, are inserted into expression vectors such that the genes are operatively linked to transcriptional and translational control sequences. In this context, the term "operatively linked" is intended to mean that an antibody gene is ligated into a vector such that transcriptional and translational control sequences within the vector serve their intended function of regulating the transcription and translation of the antibody gene. The expression vector and expression control sequences are chosen to be compatible with the expression host cell used. The antibody light chain gene and the antibody heavy chain gene can be inserted into separate vector or, more typically, both genes are inserted into the same expression vector. The antibody genes are inserted into the expression vector by standard methods (*e.g.,* ligation of complementary restriction sites on the antibody gene fragment and vector, or blunt end ligation if no restriction sites are present). Prior to insertion of the D2E7 or D2E7-related light or heavy chain sequences, the expression vector may already carry antibody constant region sequences. For example, one approach to converting the D2B7 or D2E7-related VH and VL sequences to full-length antibody genes is to insert them into expression vectors already encoding heavy chain constant and light chain constant regions, respectively, such that the VH segment is operatively linked to the CH segment(s) within the vector and the VL segment is operatively linked to the CL segment within the vector. Additionally or alternatively, the recombinant expression vector can encode a signal peptide that facilitates secretion of the antibody chain from a host cell. The antibody chain gene can be cloned into the vector such that the signal peptide is linked in-frame to the amino terminus of the antibody chain gene. The signal peptide can be an immunoglobulin signal peptide or a heterologous signal peptide (*i.e.,* a signal peptide from a non-immunoglobulin protein).

In addition to the antibody chain genes, the recombinant expression vectors of the invention carry regulatory sequences that control the expression of the antibody chain genes in a host cell. The term "regulatory sequence" is intended to includes promoters, enhancers and other expression control elements (*e*.*g*., polyadenylation signals) that control the transcription or translation of the antibody chain genes. Such regulatory sequences are described, for example, in Goeddel; Gene Expression Technology: Methods in Enzymology 185, Academic Press, San Diego, CA (1990). It will be appreciated by those skilled in the art that the design of the expression vector, including the selection of regulatory sequences may depend on such factors as the choice of the host cell to be transformed, the level of expression of protein desired, *etc.* Preferred regulatory sequences for mammalian host cell expression include viral elements that direct high levels of protein expression in mammalian cells, such as promoters and/or enhancers derived from cytomegalovirus (CMV) (such as the CMV promoter/enhancer), Simian Virus 40 (SV40) (such as the SV40 promoter/enhancer), adenovirus, (*e.g.,* the adenovirus major late promoter (AdMLP)) and polyoma. For further description of viral regulatory elements, and sequences thereof, see *e*.*g*., U.S. Patent No. 5,168,062 by Stinski, U.S. Patent No. 4,510,245 by Bell et al. and U.S. Patent No. 4,968,615 by Schaffner et al*,*

In addition to the antibody chain genes and regulatory sequences, the recombinant expression vectors of the invention may carry additional sequences, such as sequences that regulate replication of the vector in host cells (*e*.*g*., origins of replication) and selectable marker genes. The selectable marker gene facilitates selection of host cells into which the vector has been introduced (see *e*.*g*., U.S. Patents Nos. 4,399,216,4,634,665 and 5,179,017, all by Axel et al.). For example, typically the selectable marker gene confers resistance to drugs, such as G418,hygromycin or methotrexate, on a host cell into which the vector has been introduced. Preferred selectable marker genes include the dihydrofolate reductase (DHFR) gene (for use in dhfr⁻ host cells with methotrexate selection/amplification) and the *neo* gene (for G418 selection).

For expression of the light and heavy chains, the expression vector(s) encoding the heavy and light chains is transfected into a host cell by standard techniques. The various forms of the term "transfection" are intended to encompass a wide variety of techniques commonly used for the introduction of exogenous DNA into a prokaryotic or eukaryotic host cell, *e.g.,* electroporation, calcium-phosphate precipitation, DEAE-dextran transfection and the like. Although it is theoretically possible to express the antibodies of the invention in either prokaryotic or eukaryotic host cells, expression of antibodies in eukaryotic cells, and most preferably mammalian host cells, is the most preferred because such eukaryotic cells, and in particular mammalian cells, are more likely than prokaryotic cells to assemble and secrete a properly folded and immunologically active antibody. Prokaryotic expression of antibody genes has been reported to be ineffective for production of high yields of active antibody (Boss, M.A. and Wood, C. R. (1985) Immunology Today 6:12-13).

Preferred mammalian host cells for expressing the recombinant antibodies of the invention include Chinese Hamster Ovary (CHO cells) (including dhfr- CHO cells, described in Urlaub and Chasin, (1980) Proc. Natl. Acad. Sci. USA 77:4216-4220, used with a DHFR selectable marker, *e.g.,* as described in R.J. Kaufman and P.A. Sharp (1982) Mol. Biol. 159:601-621), NS0 myeloma cells, COS cells and SP2 cells. When recombinant expression vectors encoding antibody genes are introduced into mammalian host cells, the antibodies are produced by culturing the host cells for a period of time sufficient to allow for expression of the antibody in the host cells or, more preferably, secretion of the antibody into the culture medium in which the host cells are grown. Antibodies can be recovered from the culture medium using standard protein purification methods.

Host cells can also be used to produce portions of intact antibodies, such as Fab fragments or scFv molecules. It will be understood that variations on the above procedure are within the scope of the present invention. For example, it may be desirable to transfect a host cell with DNA encoding either the light chain or the heavy chain (but not both) of an antibody of this invention. Recombinant DNA technology may also be used to remove some or all of the DNA encoding either or both of the light and heavy chains that is not necessary for binding to hTNFα. The molecules expressed from such truncated DNA molecules are also encompassed by the antibodies of the invention. In addition, bifunctional antibodies may be produced in which one heavy and one light chain are an antibody of the invention and the other heavy and light chain are specific for an antigen other than hTNFα by crosslinking an antibody of the invention to a second antibody by standard chemical crosslinking methods.

In a preferred system for recombinant expression of an antibody, or antigen-binding portion thereof, of the invention, a recombinant expression vector encoding both the antibody heavy chain and the antibody light chain is introduced into dhfr-CHO cells by calcium phosphate-mediated transfection. Within the recombinant expression vector, the antibody heavy and light chain genes are each operatively linked to CMV enhancer/AdMLP promoter regulatory elements to drive high levels of transcription of the genes. The recombinant expression vector also carries a DHFR gene, which allows for selection of CHO cells that have been transfected with the vector using methotrexate selection/amplification. The selected transformant host cells are culture to allow for expression of the antibody heavy and light chains and intact antibody is recovered from the culture medium. Standard molecular biology techniques are used to prepare the recombinant expression vector, transfect the host cells, select for transformants, culture the host cells and recover the antibody from the culture medium.

Recombinant human antibodies of the invention in addition to D2E7 or an antigen binding portion thereof or D2E7-related antibodies disclosed herein can be isolated by screening of a recombinant combinatorial antibody library, preferably a scFv phage display library, prepared using human VL and VH cDNAs prepared from mRNA derived from human lymphocytes. Methodologies for preparing and screening such libraries are known in the art. In addition to commercially available kits for generating phage display libraries (*e*.*g*., the Pharmacia *Recombinant Phage Antibody System,* catalog no. 27-9400-01; and the Stratagene *SurfZAP*^{™} phage display kit, catalog no. 240612), examples of methods and reagents particularly amenable for use in generating and screening antibody display libraries can be found in, for example, Ladner et al. U.S. Patent No. 5,223,409; Kang et al. PCT Publication No. WO 92/18619; Dower et al. PCT Publication No. WO 91/17271; Winter et al. PCT Publication No. WO 92/20791; Markland et al. PCT Publication No. WO 92/15679; Breitling et al. PCT Publication No. WO 93/01288; McCafferty et al. PCT Publication No. WO 92/01047; Garrard et al. PCT Publication No. WO 92/09690; Fuchs et al. (1991) Bio/Technology 9:1370-1372; Hay et al. (1992) Hum Antibod Hybridomas 3:81-85; Huse et al, (1989) Science 246:1275-1281; McCafferty et al., Nature (1990) 348:552-554; Griffiths et al. (1993) EMBO J 12:725-734; Hawkins et al. (1992) J Mol Biol 226:889-896; Clackson et al. (1991) Nature 352:624-628; Gram et al. (1992) PNAS 89:3576-3580; Garrard et al. (1991) Bio/Technology 9:1373-1377; Hoogenboom et al. (1991) Nuc Acid Res 19:4133-4137; and Barbas et al. (1991) PNAS 88:7978-7982.

In a preferred embodiment, to isolate human antibodies with high affinity and a low off rate constant for hTNFα, a murine anti-hTNFα antibody having high affinity and a low off rate constant for hTNFα (*e*.*g*., MAK 195, the hybridoma for which has deposit number ECACC 87 050801) is first used to select human heavy and light chain sequences having similar binding activity toward hTNFα, using the epitope imprinting methods described in Hoogenboom et al., PCT Publication No. WO 93/06213. The antibody libraries used in this method are preferably scFv libraries prepared and screened as described in McCafferty et al., PCT Publication No. WO 92/01047, McCafferty et al., Nature (1990) 348:552-554; and Griffiths et al., (1993) EMBO J 12:725-734. The scFv antibody libraries preferably are screened using recombinant human TNFα as the antigen.

Once initial human VL and VH segments are selected, "mix and match" experiments, in which different pairs of the initially selected VL and VH segments are screened for hTNFα binding, are performed to select preferred VL/VH pair combinations. Additionally, to further improve the affinity and/or lower the off rate constant for hTNFα binding, the VL and VH segments of the preferred VL/VH pair(s) can be randomly mutated, preferably within the CDR3 region of VH and/or VL, in a process analogous to the *in vivo* somatic mutation process responsible for affinity maturation of antibodies during a natural immune response. This *in vitro* affinity maturation can be accomplished by amplifying VH and VL regions using PCR primers complimentary to the VH CDR3 or VL CDR3, respectively, which primers have been "spiked" with a random mixture of the four nucleotide bases at certain positions such that the resultant PCR products encode VH and VL segments into which random mutations have been introduced into the VH and/or VL CDR3 regions. These randomly mutated VH and VL segments can be rescreened for binding to hTNFα and sequences that exhibit high affinity and a low off rate for hTNFα binding can be selected.

Following screening and isolation of an anti-hTNFα antibody of the invention from a recombinant immunoglobulin display library, nucleic acid encoding the selected antibody can be recovered from the display package (*e*.*g*., from the phage genome) and subcloned into other expression vectors by standard recombinant DNA techniques. If desired, the nucleic acid can be further manipulated to create other antibody forms of the invention (*e*.*g*., linked to nucleic acid encoding additional immunoglobulin domains, such as additional constant regions). To express a recombinant human antibody isolated by screening of a combinatorial library, the DNA encoding the antibody is cloned into a recombinant expression vector and introduced into a mammalian host cells, as described in further detail in Section II above.

The antibodies and antibody-portions of the invention and a drug that is useful for treating an autoimmune disease can be incorporated separately or together into pharmaceutical compositions suitable for administration to a subject for the methods described herein. Typically, the pharmaceutical composition comprises an antibody (or antibody portion) of the invention and/or one or more other drugs that are useful in treating an autoimmune disease and a pharmaceutically acceptable carrier. As used herein, "pharmaceutically acceptable carrier" includes any and all solvents, dispersion media, coatings, antibacterial and antifungal agents, isotonic and absorption delaying agents, and the like that ate physiologically compatible and are suitable for administration to a subject for the methods described herein. Examples of pharmaceutically acceptable carriers include one or more of water, saline, phosphate buffered saline, dextrose, glycerol, ethanol and the like, as well as combinations thereof. In many cases, it will be preferable to include isotonic agents, for example, sugars, polyalcohols such as mannitol, sorbitol, or sodium chloride in the composition. Pharmaceutically acceptable carriers may further comprise minor amounts of auxiliary substances such as wetting or emulsifying agents, preservatives or buffers, which enhance the shelf life or effectiveness of the antibody or antibody portion.

The compositions of this invention may be in a variety of forms. These include, for example, liquid, semi-solid and solid dosage forms, such as liquid solutions (*e*.*g*., injectable and infusible solutions), dispersions or suspensions, tablets, pills, powders, liposomes and suppositories. The preferred form depends on the intended mode of administration and therapeutic application. Typical preferred compositions are in the form of injectable or infusible solutions, such as compositions similar to those used for passive immunization of humans with other antibodies. The preferred mode of administration is parenteral (*e*.*g*., intravenous, subcutaneous, intraperitoneal, intramuscular). In a preferred embodiment, the pharmaceutical composition comprising the antibody is administered by injection. In another preferred embodiment, the antibody is administered by intramuscular injection. In a particularly preferred embodiment, the antibody is administered by subcutaneous injection.

Pharmaceutical compositions typically must be sterile and stable under the conditions of manufacture and storage. The composition can be formulated as a solution, microemulsion, dispersion, liposome, or other ordered structure suitable to high drug concentration. Sterile injectable solutions can be prepared by incorporating the active compound (*i*.*e*., antibody or antibody portion) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying that yields a powder of the active ingredient plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption, for example, monostearate salts and gelatin.

The antibodies and antibody-portions of the present invention can be administered by a variety of methods known in the art, although for many therapeutic applications, the preferred route/mode of administration is subcutaneous injection. As will be appreciated by the skilled artisan, the route and/or mode of administration will vary depending upon the desired results. In certain embodiments, the active compound may be prepared with a carrier that will protect the compound against rapid release, such as a controlled release formulation, including implants, transdermal patches, and microencapsulated delivery systems. Biodegradable, biocompatible polymers can be used, such as ethylene vinyl acetate, polyethylene glycol (PEG), polyanhydrides, polyglycolic acid, collagen, polyorthoesters, and polylactic acid. Many methods for the preparation of such formulations are patented or generally known to those skilled in the art. See, *e.g.,* Sustained and Controlled Release Drug Delivery Systems, J.R. Robinson, ed., Marcel Dekker, Inc., New York, 1978.

In certain embodiments, an antibody or antibody portion of the invention may be orally administered, for example, with an inert diluent or an assimilable edible carrier. The compound (and other ingredients, if desired) may also be enclosed in a hard or soft shell gelatin capsule, compressed into tablets, or incorporated directly into the subject's diet. For oral therapeutic administration, the compounds may be incorporated with excipients and used in the form of ingestible tablets, buccal tablets, troches, capsules, elixirs, suspensions, syrups, wafers, and the like. To administer a compound of the invention by other than parenteral administration, it may be necessary to coat the compound with, or co-administer the compound with, a material to prevent its inactivation.

Supplementary active compounds can also be incorporated into the compositions. In certain embodiments, an antibody or antibody portion of the invention is coformulated with and/or coadministered with one or more additional therapeutic agents. For example, an anti- hTNFα antibody or antibody portion of the invention may be coformulated and/or coadministered with one or more DMARD or one or more NSAID or one or more additional antibodies that bind other targets (*e.g.,* antibodies that bind other cytokines or that bind cell surface molecules), one or more cytokines, soluble TNFα receptor (see *e*.*g.,* PCT Publication No. WO 94/06476) and/or one or more chemical agents that inhibit hTNFα production or activity (such as cyclohexane-ylidene derivatives as described in PCT Publication No. WO 93/19751) or any combination thereof. Furthermore, one or more antibodies of the invention may be used in combination with two or more of the foregoing therapeutic agents. Such combination therapies may advantageously utilize lower dosages of the administered therapeutic agents, thus avoiding possible side effects, complications or low level of response by the patient associated with the various monotherapies.

The pharmaceutical compositions of the invention may include a "therapeutically effective amount" or a "prophylactically effective amount" of an antibody or antibody portion of the invention and/or other drug(s). A "therapeutically effective amount" refers to an amount effective, at dosages and for periods of time necessary, to achieve the desired therapeutic result. A therapeutically effective amount may vary according to factors such as the disease state, age, sex, and weight of the individual, and the ability of the antibody or antibody portion or other drug that is useful for treating an autoimmune disease to elicit a desired response in the individual. A therapeutically effective amount is also one in which side effects of the antibody or antibody portion or the other drug(s) are outweighed by the therapeutically beneficial effects. A "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount.

Dosage regimens may be adjusted to provide the optimum desired response (*e*.*g*., a therapeutic or prophylactic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic or prophylactic effect to be achieved, and (b) the limitations inherent in the art of compounding such an active compound for the treatment of sensitivity in individuals.

An exemplary, non-limiting range for a therapeutically or prophylactically effective amount of an antibody or antibody portion of the invention is 10-100 mg, more preferably 20-80 mg and most preferrably about 40 mg. It is to be noted that dosage values may vary with the type and severity of the condition to be alleviated. It is to be further understood that for any particular subject, specific dosage regimens should be adjusted over time according to the individual need and the professional judgment of the person administering or supervising the administration of the compositions, and that dosage ranges set forth herein are exemplary only and are not intended to limit the scope or practice of the claimed composition.

Non-limiting examples of therapeutic agents for rheumatoid arthritis with which an antibody, or antibody portion, of the invention can be combined include the following: non-steroidal anti-inflammatory drug(s) (NSAIDs); cytokine suppressive anti-inflammatory drug(s) (CSAIDs); CDP-571/BAY-10-3356 (humanized anti-TNFα antibody; Celltech/Bayer); cA2 (chimeric anti-TNFα antibody; Centocor); 75 kdTNFR-IgG (75 kD TNF receptor-IgG fusion protein; Immunex; see *e.g.,* Arthritis & Rheumatism (1994) Vol. 37, S295; J. Invest. Med. (1996) Vol. 44, 235A); 55 kdTNFR-IgG (55 kD TNF receptor-IgG fusion protein; Hoffmann-LaRoche); IDEC-CE9.1/SB 210396 (non-depleting primatized anti-CD4 antibody; IDEC/SmithKline; see *e.g.,* Arthritis & Rheumatism (1995) Vol. 38, S185); DAB 486-IL-2 and/or DAB 389-IL-2 (IL-2 fusion proteins; Seragen; see *e.g.,* Arthritis & Rheumatism (1993) Vol. 36, 1223); Anti-Tac (humanized anti-IL-2Rα; Protein Design Labs/Roche); IL-4 (anti-inflammatory cytokine; DNAX/Schering); IL-10 (SCH 52000; recombinant IL-10, anti-inflammatory cytokine; DNAX/Schering); IL-4; IL-10 and/or IL-4 agonists (*e.g.,* agonist antibodies); IL-1RA (IL-1 receptor antagonist; Synergen/Amgen); TNF-bp/s-TNFR (soluble TNF binding protein; see *e.g.,* Arthritis & Rheumatism (1996) Vol. 39, No. 9 (supplement), S284; Amer. J. Physiol. - Heart and Circulatory Physiology (1995) Vol. 268, pp. 37-42); R973401 (phosphodiesterase Type IV inhibitor; see *e.g.,* Arthritis & Rheumatism (1996) Vol. 39, No. 9 (supplement), S282); MK-966 (COX-2 Inhibitor; see *e.g.,* Arthritis & Rheumatism (1996) Vol. 39, No. 9 (supplement), S81); Iloprost (see *e.g.,* Arthritis & Rheumatism (1996) Vol. 39, No. 9 (supplement), S82); methotrexate; thalidomide (see *e.g.,* Arthritis & Rheumatism (1996) Vol. 39, No. 9 (supplement), S282) and thalidomide-related drugs (*e*.*g*., Celgen); leflunomide (anti-inflammatory and cytokine inhibitor; see *e.g.,* Arthritis & Rheumatism (1996) Vol. 39, No. 9 (supplement), S131; Inflammation Research (1996) Vol. 45, pp. 103-107); tranexamic acid (inhibitor of plasminogen activation; see *e.g.,* Arthritis & Rheumatism (1996) Vol. 39, No. 9 (supplement), S284); T-614 (cytokine inhibitor; see *e.g.,* Arthritis & Rheumatism (1996) Vol. 39, No. 9 (supplement), S282); prostaglandin E1 (see *e.g.,* Arthritis & Rheumatism (1996) Vol. 39, No. 9 (supplement), S282); Tenidap (non-steroidal anti-inflammatory drug; see *e.g.,* Arthritis & Rheumatism (1996) Vol. 39, No. 9 (supplement), S280); Naproxen (non-steroidal anti-inflammatory drug; see *e.g.,* Neuro Report (1996) Vol. 7, pp. 1209-1213); Meloxicam (non-steroidal anti-inflammatory drug); Ibuprofen (non-steroidal anti-inflammatory drug); Piroxicam (non-steroidal anti-inflammatory drug); Diclofenac (non-steroidal anti-inflammatory drug); Indomethacin (non-steroidal anti-inflammatory drug); Sulfasalazine (see *e*.*g*., Arthritis & Rheumatism (1996) Vol. 39, No. 9 (supplement), S281); Azathioprine (see *e.g.,* Arthritis & Rheumatism (1996) Vol. 39, No. 9 (supplement), S281); ICE inhibitor (inhibitor of the enzyme interleukin-1β converting enzyme); zap-70 and/or Ick inhibitor (inhibitor of the tyrosine kinase zap-70 or lck); VEGF inhibitor and/or VEGF-R inhibitor (inhibitors of vascular endothelial cell growth factor or vascular endothelial cell growth factor receptor; inhibitors of angiogenesis); corticosteroid anti-inflammatory drugs (*e*.*g*., SB203580); TNF-convertase inhibitors; anti-IL-12 antibodies; interleukin-11 (see *e*.*g*., Arthritis & Rheumatism (1996) Vol. 39, No.9 (supplement), S296); interleukin-13 (see *e*.*g*., Arthritis & Rheumatism (1996) Vol. 39, No. 9 (supplement), S308); interleukin-17 inhibitors (see *e.g.,* Arthritis & Rheumatism (1996) Vol. 39, No. 9 (supplement), S120); gold; penicillamine; chloroquine; hydroxychloroquine; chlorambucil; cyclophosphamide; cyclosporine; total lymphoid irradiation; anti-thymocyte globulin; anti-CD4 antibodies; CD5-toxins; orally-administered peptides and collagen; lobenzarit disodium; Cytokine Regulating Agents (CRAs) HP228 and HP466 (Houghten Pharmaceuticals, Inc.); ICAM-1 antisense phosphorothioate oligodeoxynucleotides (ISIS 2302; Isis Pharmaceuticals, Inc.); soluble complement receptor 1 (TP10; T Cell Sciences, Inc.); prednisone; orgotein; glycosaminoglycan polysulphate; minocycline; anti-IL2R antibodies; marine and botanical lipids (fish and plant seed fatty acids; see *e*.*g*., DeLuca et al. (1995) Rheum. Dis. Clin. North Am. 21:759-777); auranofin; phenylbutazone; meclofenamic acid; flufenamic acid; intravenous immune globulin; zileuton; mycophenolic acid (RS-61443); tacrolimus (FK-506); sirolimus (rapamycin); amiprilose (therafectin); cladribine (2-chlorodeoxyadenosine); and azaribine.

Non-limiting examples of therapeutic agents for inflammatory bowel disease with which an antibody, or antibody portion, of the invention can be combined include the following: budenoside; epidermal growth factor; corticosteroids; cyclosporin, sulfasalazine; aminosalicylates; 6-mercaptopurine; azathioprine; metronidazole; lipoxygenase inhibitors; mesalamine; olsalazine; balsalazide; antioxidants; thromboxane inhibitors; IL-1 receptor antagonists; anti-IL-1β monoclonal antibodies; anti-IL-6 monoclonal antibodies; growth factors; elastase inhibitors; pyridlnyl-imidazole compounds; CDP-571/BAY-10-3356 (humanized anti-TNFα antibody; Celltech/Bayer); cA2 (chimeric anti-TNFα, antibody; Centocor); 75 kdTNFR-IgG (75 kD TNF receptor-IgG fusion protein; Immunex; see *e*.*g*., Arthritis & Rheumatism (1994) Vol. 37, S295; J. Invest. Med. (1996) Vol. 44, 235A); 55 kdTNFR-IgG (55 kD TNF receptor IgG fusion protein; Hoffmann-LaRoche); interleukin-10 (SCH 52000; Schering Plough); IL-4; IL-10 and/or IL-4 agonists (*e*.*g*., agonist antibodies); interleukin-11; glucuronide- or dextran-conjugated prodrugs of prednisolone, dexamethasone or budesonide; ICAM-1 antisense phosphorothioate oligodeoxynucleotides (ISIS 2302; Isis Pharmaceuticals, Inc.); soluble complement receptor 1 (TP10; T Cell Sciences, Inc.); slow-release mesalazine; methotrexate; antagonists of Platelet Activating Factor (PAF); ciprofloxacin; and lignocaine.

Nonlimiting examples of therapeutic agents for multiple sclerosis with which an antibody, or antibody portion, of the invention can be combined include the following: corticosteroids; prednisolone; methylprednisolone; azathioprine; cyclophosphamide; cyclosporine; methotrexate; 4-aminopyridine; tizanidine; interferon-β1a (Avonex^{™}; Biogen); interferon-β1b (Betaseron^{™}; Chiron/Berlex); Copolymer 1 (Cop-1; Copaxone^{™}; Teva Pharmaceutical Industries, Inc.); hyperbatic oxygen; intravenous immunoglobulin; clabribine; CDP-571/BAY-10-3356 (humanized anti-TNFα antibody; Celltech/Bayer); cA2 (chimeric anti-TNFα antibody; Centocor); 75 kdTNFR-IgG (75 kD TNF receptor-IgG fusion protein; Immunex; see *e.g.,* Arthritis & Rheumatism (1994) Vol. 37, S295; J. Invest. Med. (1996) Vol. 44, 235A); 55 kdTNFR-IgG (55 kD TNF receptor-IgG fusion protein; Hoffmann-LaRoche); IL-10; IL-4; and IL-10 and/or IL-4 agonists (*e*.*g*., agonist antibodies).

Nonlimiting examples of therapeutic agents for sepsis with which an antibody, or antibody portion, of the invention can be combined include the following: hypertonic saline solutions; antibiotics; intravenous gamma globulin; continuous hemofiltration; carbapenems (*e.g.,* meropenem); antagonists of cytokines such as TNFα, IL-1β, IL-6 and/or TL-8; CDP-571/BAY-10-3356 (humanized anti-TNFα antibody; Celltech/Bayer); cA2 (chimeric anti-TNFα antibody; Centocor); 75 kdTNFR-IgG (75 kD TNF receptor-IgG fusion protein; Immunex; see *e.g.,* Arthritis & Rheumatism (1994) Vol. 37, S295 J. Invest. Med. (1996) Vol. 44, 235A); 55 kdTNFR-IgG (55 kD TNF receptor-IgG fusion protein; Hoffmann-LaRoche); Cytokine Regulating Agents (CRAs) HP228 and HP466 (Houghten Pharmaceuticals, Inc.); SK&F 107647 (low molecular peptide; SmithKline Beecham); tetravalent guanylhydrazone CNI-1493 (Picower Institute); Tissue Factor Pathway Inhibitor (TFPI; Chiron); PHP (chemically modified hemoglobin; APEX Bioscience); iron chelators and chelates, including diethylenetriamine pentaacetic acid - iron (III) complex (DTPA iron (III); Molichem Medicines); lisofylline (synthetic small molecule methylxanthine; Cell Therapeutics, Inc.); PGG-Glucan (aqeuous soluble β1,3glucan; Alpha-Beta Technology); apolipoprotein A-1 reconstituted with lipids; chiral hydroxamic acids (synthetic antibacterials that inhibit lipid A biosynthesis); anti-endotoxin antibodies; E5531 (synthetic lipid A antagonist; Eisai America, Inc.); rBPI₂₁ (recombinant N-terminal fragment of human Bactericidal/Permeability-Increasing Protein); and Synthetic Anti-Endotoxin Peptides (SAEP; BiosYnth Research Laboratories);
Nonlimiting examples of therapeutic agents for adult respiratory distress syndrome (ARDS) with which an antibody, or antibody portion, of the invention can be combined include the following: anti-IL-8 antibodies; surfactant replacement therapy; CDP-571/BAY-10-3356 (humanized anti-TNFα antibody; Celltech/Bayer); cA2 (chimeric anti-TNFα antibody; Centocor); 75 kdTNFR-IgG (75 kD TNF receptor-IgG fusion protein; Immunex; see *e*.*g*., Arthritis & Rheumatism (1994) Vol. 37, 5295; J. Invest. Med. (1996) Vol. 44, 235A); and 55 kdTNFR-IgG (55 kD TNF receptor-TgG fusion protein; Hoffmann-LaRoche).

The uses of a combination of an anti-TNFα antibody with one or more other drugs will depend upon whether it is a disorder which is affected by the inhibition of TNFα. As used herein, the term "a disorder in which the administration of an anti-TNFα antibody is beneficial " is intended to include diseases and other disorders in which the presence of TNFα in a subject suffering from the disorder has been shown to be or is suspected of being either responsible for the pathophysiology of the disorder or a factor that contributes to a worsening of the disorder, or where it has been shown that another anti-TNFα antibody or a biologically active portion thereof has been successfully used to treat the disease. Accordingly, a disorder in which TNFα activity is detrimental is a disorder in which inhibition of TNFα activity is expected to alleviate the symptoms and/or progression of the disorder. Such disorders may be evidenced, for example, by an increase in the concentration of TNFα in a biological fluid of a subject suffering from the disorder (*e*.*g*., an increase in the concentration of TNFα in serum, plasma, synovial fluid, *etc.* of the subject), which can be detected, for example, using an anti-TNFα antibody as described above. There are numerous examples of disorders in which TNFα activity is detrimental:

### A. Sepsis

Tumor necrosis factor has an established role in the pathophysiology of sepsis, with biological effects that include hypotension, myocardial suppression, vascular leakage syndrome, organ necrosis, stimulation of the release of toxic secondary mediators and activation of the clotting cascade (see *e.g.,* Tracey, K,J, and Cerami, A. (1994) Annu. Rev. Med. 45:491-503; Russell, D and Thompson, R.C. (1993) Curr. Opin. Biotech. 4:714-721). Accordingly, the human antibodies, and antibody portions, of the invention can be used to treat sepsis in any of its clinical settings, including septic shock, endotoxic shock, gram negative sepsis and toxic shock syndrome. Preferably the antibodies, and antibody portions, are useful in conjunction with another drug that is useful in the treatment of sepsis. More preferred is where the antibody is D2E7. Even more preferred is where D2E7 is administered subcutaneously biweekly at a dosage of 40 mg along with one or more other drugs useful in treating sepsis.

Furthermore, to treat sepsis, an anti-hTNFα antibody, or antibody portion, of the invention can be coadministered with one or more additional therapeutic agents that may further alleviate sepsis, such as an interleukin-1 inhibitor (such as those described in PCT Publication Nos. WO 92/16221 and WO 92/17583), the cytokine interleukin-6 (see *e.g.,* PCT Publication No. WO 93/11793) or an antagonist of platelet activating factor (see *e*.*g*., European Patent Application Publication No. EP 374 510).

Additionally, in a preferred embodiment, an anti-TNFα antibody or antibody portion of the invention is administered to a human subject within a subgroup of sepsis patients having a serum or plasma concentration of IL-6 above 500 pg/ml, and more preferably 1000 pg/ml, at the time of treatment (see PCT Publication No. WO 95/20978 by Daum, L., et al.).

### B. Autoimmune Diseases

Tumor necrosis factor has been implicated in playing a role in the pathophysiology of a variety of autoimmune diseases, For example, TNFα has been implicated in activating tissue inflammation and causing joint destruction in rheumatoid arthritis (see e,g., Tracey and Cerami, *supra*; Arend, W.P. and Dayer, J-M. (1995) Arth. Rheum. 38:151-160; Fava, R.A., et al. (1993) Clin. Exp. Immunol. 94:261-266). TNFα also has been implicated in promoting the death of islet cells and in mediating insulin resistance in diabetes (see *e.g.,* Tracey and Cerami, *supra*; PCT Publication No. WO 94/08609). TNFα also has been implicated in mediating cytotoxicity to oligodendrocytes and induction of inflammatory plaques in multiple sclerosis (see *e.g.,* Tracey and Cerami, *supra*). Chimeric and humanized murine anti-hTNFα antibodies have undergone clinical testing for treatment of rheumatoid arthritis (see *e.g.,* Elliott, M.J., et al. (1994) Lancet 344:1125-1127; Elliot, M.J., et al. (1994) Lance 344:1105-1110; Rankin, E.C., et al. (1995) Br. J. Rheumatol. 34:334-342).

The human antibodies, and antibody portions of the invention can be used to treat autoimmune diseases. Preferably the antibodies, and antibody portions, are useful in conjunction with another drug that is useful in the treatment of an autoimmune disease. More preferred is where the antibody is D2E7. Even more preferred is where D2E7 is administered subcutaneously biweekly at a dosage of 40 mg along with one or more other drugs useful in treating an autoimmune disease. In particular those associated with inflammation, including rheumatoid arthritis, rheumatoid spondylitis, osteoarthritis and gouty arthritis, allergy, multiple sclerosis, autoimmune diabetes, autoimmune uveitis and nephrotic syndrome. Typically, the antibody, or antibody portion, is administered systemically, although for certain disorders, local administration of the antibody or antibody portion at a site of inflammation may be beneficial (*e*.*g*., local administration in the joints in rheumatoid arthritis or topical application to diabetic ulcers, alone or in combination with a cyclohexane-ylidene derivative as described in PCT Publication No. WO 93/19751).

### C. Infectious Diseases

Tumor necrosis factor has been implicated in mediating biological effects observed in a variety of infectious diseases. For example, TNFα has been implicated in mediating brain inflammation and capillary thrombosis and infarction in malaria (see *e*.*g*., Tracey and Cerami, *supra*). TNFα also has been implicated in mediating brain inflammation, inducing breakdown of the blood-brain barrier, triggering septic shock syndrome and activating venous infarction in meningitis (see *e*.*g*., Tracey and Cerami, *supra*). TNFα also has been implicated in inducing cachexia, stimulating viral proliferation and mediating central nervous system injury in acquired immune deficiency syndrome (AIDS) (see *e*.*g*., Tracey and Cerami, *supra*). Accordingly, the antibodies, and antibody portions, of the invention, can be used in the treatment of infectious diseases. Preferably the antibodies, and antibody portions, are useful in conjunction with another drug that is useful in the treatment of an infectious disease. More preferred is where the antibody is D2E7. Even more preferred is where D2E7 is administered subcutaneously biweekly at a dosage of 40 mg along with one or more other drugs useful in treating an infectious disease. In particular, bacterial meningitis (see *e*.*g*., European Patent Application Publication No. EP 585 705), cerebral malaria, AIDS and AIDS-related complex (ARC) (see *e*.*g*., European Patent Application Publication No. EP 230 574), as well as cytomegalovirus infection secondary to transplantation (see *e*.*g*., Fietze, E., et al. (1994) Transplantation 58:675-680). The antibodies, and antibody portions, of the invention, also can be used to alleviate symptoms associated with infectious diseases, including fever and myalgias due to infection (such as influenza) and cachexia secondary to infection (*e*.*g*., secondary to AIDS or ARC).

### D. Transplantation

Tumor necrosis factor has been implicated as a key mediator of allograft rejection and graft versus host disease (GVHD) and in mediating an adverse reaction that has been observed when the rat antibody OKT3, directed against the T cell receptor CD3 complex, is used to inhibit rejection of renal transplants (see *e*.*g*., Tracey and Cerami, *supra*; Eason, J.D., et al. (1995) Transplantation 59;300-305; Suthanthiran, M. and Strom, T,B. (1994) New Engl. J. Med. 331:365-375). Accordingly, the antibodies, and antibody portions, of the invention, can be used to inhibit transplant rejection, including rejections of allografts and xenografts and to inhibit GVHD. Preferably the antibodies, and antibody portions, are useful in conjunction with another drug that is useful in the treatment of transplant rejection. More preferred is where the antibody is D2E7. Even more preferred is where D2E7 is administered subcutaneously biweekly at a dosage of 40 mg along with one or more other drugs useful in treating transplant rejection. For example, in one embodiment, an antibody or antibody portion of the invention is used in combination with OKT3 to inhibit OKT3-induced reactions. In another embodiment, an antibody or antibody portion of the invention is used in combination with one or more antibodies directed at other targets involved in regulating immune responses, such as the cell surface molecules CD25 (interleukin-2 receptor-α), CD11a (LFA-1), CD54 (ICAM-1), CD4, CD45, CD28/CTLA4, CD80 (B7-1) and/or CD86 (B7-2). In yet another embodiment, an antibody or antibody portion of the invention is used in combination with one or more general immunosuppressive agents, such as cyclosporin A or FK506.

### E. Malignancy

Tumor necrosis factor has been implicated in inducing cachexia, stimulating tumor growth, enhancing metastatic potential and mediating cytotoxicity in malignancies (see *e.g.,* Tracey and Cerami, *supra*)*.* Accordingly, the antibodies, and antibody portions, of the invention, can be used in the treatment of malignancies, to inhibit tumor growth or metastasis and/or to alleviate cachexia secondary to malignancy. Preferably the antibodies, and antibody portions, are useful in conjunction with another drug that is useful in the treatment of malignancies, to inhibit tumor growth or metastasis and/or to alleviate cachexia secondary to malignancy. More preferred is where the antibody is D2E7. Even more preferred is where D2E7 is administered subcutaneously biweekly at a dosage of 40 mg along with one or more other drugs useful in treating a malignancy, to inhibit tumor growth or metastasis and/or to alleviate cachexia secondary to malignancies. The antibody, or antibody portion, may be administered systemically or locally to the tumor site.

### F. Pulmonary Disorders

Tumor necrosis factor has been implicated in the pathophysiology of adult respiratory distress syndrome, including stimulating leukocyte-endothelial activation, directing cytotoxicity to pneumocytes and inducing vascular leakage syndrome (see *e*.*g*., Tracey and Cerami, *supra*). Accordingly, the antibodies, and antibody portions, of the invention, can be used to treat various pulmonary disorders. Preferably the antibodies, and antibody portions, are useful in conjunction with another drug that is useful in the treatment of a pulmonary disorder. More preferred is where the antibody is D2E7. Even more preferred is where D2E7 is administered subcutaneously biweekly at a dosage of 40 mg along with one or more other drugs useful in treating a pulmonary disorder. In particular, adult respiratory distress syndrome (see *e*.*g*., PCT Publication No. WO 91/04054), shock lung, chronic pulmonary inflammatory disease, pulmonary sarcoidosis, pulmonary fibrosis and silicosis. The antibody, or antibody portion, may be administered systemically or locally to the lung surface, for example as an aerosol.

### G. Intestinal Disorders

Tumor necrosis factor has been implicated in the pathophysiology of inflammatory bowel disorders (see *e.g.,* Tracy, K.J., et al. (1986) Science 234:470-474; Sun, X-M., et al. (1988) J. Clin, Invest. 81:1328-1331; MacDonald, T.T., et al. (1990) Clin. Exp. Immunol. 81:301-305). Chimeric murine anti-hTNFα antibodies have undergone clinical testing for treatment of Crohn's disease (van Dullemen, H.M., et al. (1995) Gastroenterology 109:129-135). The human antibodies, and antibody portions, of the invention, also can be used to treat intestinal disorders. Preferably the antibodies, and antibody portions, are useful in conjunction with another drug that is useful in the treatment of an intestinal disorder. More preferred is where the antibody is D2E7. Even more preferred is where D2E7 is administered subcutaneously biweekly at a dosage of 40 mg along with one or more other drugs useful in treating an intestinal disorder. In particular, idiopathic inflammatory bowel disease, which includes two syndromes, Crohn's disease and ulcerative colitis.

### H. Cardiac Disorders

The antibodies, and antibody portions, of the invention, also can be used to treat various cardiac disorders, including ischemia of the heart (see *e*.*g*., European Patent Application Publication No. EP 453 898) and heart insufficiency (weakness of the heart muscle) (see *e*.*g*., PCT Publication No. WO 94/20139). Preferably the antibodies, and antibody portions, are useful in conjunction with another drug that is useful in the treatment of a cardiac disorder. More preferred is where the antibody is D2E7. Even more preferred is where D2E7 is administered subcutaneously biweekly at a dosage of 40 mg along with one or more other drugs useful in treating a cardiac disorder.

### I. Neurological Disorders

The antibodies, and antibody portions, of the invention can be used to treat a neurological disorder. Preferably the antibodies, and antibody portions, are useful in conjunction with another drug that is useful in the treatment of a neurological disorder. More preferred is where the antibody is D2E7. Even more preferred is where D2E7 is administered subcutaneously biweekly at a dosage of 40 mg along with one or more other drugs useful in treating a neurological disorder.

Experimental evidence has shown that excessive levels of TNF are released by injury to neuronal tissue. Accordingly, the use of TNF antagonists will result in amelioration of these neurological conditions. Neurological disorders due to demyelinating disease (e.g. multiple sclerosis), immune disease, inflammation, trauma, or compression, occur in different clinical forms depending upon the anatomic site and the cause and natural history of the physiological problem. Common to all of these disorders is the fact that they can cause permanent neurological damage, that damage can occur rapidly and be irreversible, and that current treatment of these conditions is unsatisfactory, often requiring surgery and/or the use of pharmacologic agents, which are often not completely successful. These neurological conditions include acute spinal cord trauma, spinal cord compression, spinal cord hematoma, cord contusion (these cases are usually traumatic, such as motorcycle accidents or sports injuries); nerve compression, the most common condition being a herniated disc causing sciatic nerve compression, neuropathy, and pain; but also including cervical disc herniation, causing nerve compression in the neck; carpal tunnel syndrome (non-RA); acute or chronic spinal cord compression from cancer (this is usually due to metastases to the spine, such as from prostate, breast or lung cancer); autoimmune disease of the nervous system; and demyelinating diseases, the most common condition being multiple sclerosis.

An example of another drug that is useful in treating a neurological disorder are steroid drugs such as cortisone that are used to treat the aforementioned neurological problems and conditions. Further examples of pharmacologic chemical substances, compounds and agents which are used for the treatment of neurological disorders, trauma, injuries and compression having various organic structures and metabolic functions are disclosed in, for example, U.S. Pat. Nos. 5,756,482 and 5,574,022, which are incorporated herein by reference in their entirety, that disclose methods of attenuating physical damage to the nervous system and to the spinal cord after injury using steroid hormones or steroid precursors such as pregnenolone, and pregnenolone sulfate in conjunction with a non-steroidal anti- inflammatory substance such as indomethacin.

### J. Others

The antibodies, and antibody portions, of the invention, also can be used to treat various other disorders in which TNFα activity is detrimental. Examples of other diseases and disorders in which TNFα activity has been implicated in the pathophysiology, and thus which can be treated using an antibody, or antibody portion, of the invention, include inflammatory bone disorders and bone resorption disease (see *e*.*g*., Bertolini, D.R., et al. (1986) Nature 319:516-518; Konig, A., et al. (1988) J. Bone Miner, Res.3:621-627; Lerner, U.H. and Ohlin, A. (1993) J. Bone Miner. Res. 8:147-155; and Shankar, G, and Stern, P.H. (1993) Bone 14:871-876), hepatitis, including alcoholic hepatitis (see *e.g.,* McClain, C.J. and Cohen, D.A. (1989) Hepatology 9:349-351; Felver, M.E., et al. (1990) Alcohol. Clin. Exp. Res. 14:255-259; and Hansen, J., et al. (1994) Hepatology 20:461-474) and viral hepatitis (Sheron, N., et al. (1991) J. Hepatol. 12:241-245; and Hussain, M.J., et al. (1994) J. Clin. Pathol. 47:1112-1115), coagulation disturbances (see *e.g.,* van der Poll, T., et al. (1990) N. Engl. J. Med. 322:1622-1627; and van der Poll, T., et al. (1991) Prog. Clin. Biol. Res. 367:55-60), burns (see *e*.*g*., Git-ozr, B.P., et al. (1994) Am. J. Physiol. 267:H118-124; and Liu, X.S., et al. (1994) Burns 20:40-44), reperfusion injury (see *e.g.,* Scales, W.E., et al. (1994) Am. J. Physiol. 267:G1122-1127; Serrick, C., et al. (1994) Transplantation 58:1158-1162; and Yao, Y.M., et al. (1995) Resuscitation 29:157-168), keloid formation (see *e.g.,* McCauley, R.L., et al. (1992) J. Clin. Immunol. 12:300-308), scar tissue formation and pyrexia. Preferably the antibodies, and antibody portions, are useful in conjunction with another drug that is useful in treating the respective disorder or disease. More preferred is where the antibody is D2E7. Even more preferred is where D2E7 is administered subcutaneously biweekly at a dosage of 40 mg along with one or more other drugs useful in treating the respective disorder or disease.

This invention is further illustrated by the following examples which should not be construed as limiting. The contents of all references, patents and published patent applications cited throughout this application are hereby incorporated by reference. Where combinations of the foregoing and following disclosure are applicable and possible, such combinations are within the scope of the present invention even if the combiantion is not stated *ipsis verbis.*

### Example

A study was designed and conducted to investigate the safety and efficacy of D2E7 40 mg administered every other week to patients with RA whose disease was not adequately treated with their current anti-rheumatic therapies (including accepted DMARDs, NSAIDs, or steroids); and was a multicenter, randomized, double-blind, placebo-controlled study in which D2E7 was subcutaneously (sc) administered every other week for up to 24 weeks at a dosage of 40 mg to patients with rheumatoid arthritis (RA) whose disease was not adequately treated with their current anti-rheumatic therapies. Anti-rheumatic therapies permitted for use during the study included accepted disease-modifying anti-rheumatic drugs (DMARDs), non-steroidal anti-inflammatory drugs (NSAIDs), or steroids. Despite using anti-rheumatic therapies, patients must have had active disease documented at both the screening and baseline visits. Doses of DMARDs, as well as concomitant prednisone (≤10 mg daily) and NSAIDs, were required to be stable for at least 28 days prior to screening. Additionally, patients using azathioprine and/or cyclosporine discontinued these therapies and underwent a 28-day washout period before screening. At the baseline visit, patients were randomized to D2E7 or placebo and this signified the start of the 24-week placebo-controlled period. Patients were examined at Weeks 2, 4, 8, 12, 16, 20 and 24 of the study. Patients who failed to meet or maintain an American College of Rheumatology 20% improvement (ACR20) response were allowed a single increase in dosage of their DMARD and/or steroid therapy, treatment with another DMARD after 3 months of study participation, or further dose adjustments following consultation with the Knoll medical monitor.

Seven hundred fifty (750) patients were enrolled, 636 patients were randomized, 578 patients completed and 636 patients were analyzed.

In contrast, all randomized patients were included in the demographic and safety analyses.

Patients had a confirmed diagnosis of RA (as defined by the 1987-revised ACR criteria) for at least 3 months and were in ACR functional class I, II, or III. Patients were inadequately treated with their current anti-rheumatic therapies and had active RA, defined as ≥6 swollen joints and ≥9 tender joints. Additionally, patients using glucocorticoids were allowed into the study if the dose was equivalent to ≤10 mg/day prednisone and had remained unchanged for at least 28 days.

D2E7 was supplied in 2-mL glass vials (the concentration of D2E7 in each vial was 25 mg/mL (ie, 40 mg/1.6mL)), each containing 40 mg D2E7/1.6 mL solution for injection. D2E7 was manufactured and supplied by Ebewe Arzneimittel GmbH, in Unterach, Austria. McKesson, HBOC Biosciences, in Rockville, MD, USA, packaged, labeled, stored and distributed D2E7 to the investigators at each site for this study. D2E7 was self-administered as a single sc injection of 40 mg every other week for a period of 24 weeks. The concentration of study drug was 25mg/mL. Placebo/1.6 mL (citrate-phosphate buffer solution without D2E7) was self-administered as a single sc injection every other week for a period of 24 weeks.

Patients continued to receive their pre-study dose of anti-rheumatic therapies. Anti-rheumatic therapies permitted for use during the study included DMARDs (hydroxychloroquine, leflunomide, methotrexate, parenteral gold, oral gold and sulfasalazine, or any combination of these or other DMARDs), NSAIDs and steroids. Doses of these DMARDs as well as concomitant prednisone (≤10 mg daily) and NSAIDs must have been stable for at least 28 days prior to screening.

All efforts were made to keep the patient in the study during the 24-week placebo-controlled period. Since this protocol was designed to reflect current clinical practice, the following adjunctive treatments and dose adjustments were allowed:

A maximum of three intra-articular steroid injections were permitted during the first 3 months of the study; the injected joint(s) were not assessed during joint examinations for 28 days following each injection.

Due to tolerability issues, the dose of background DMARD and/or steroid or NSAID therapies could be adjusted once during the study; further dose adjustments were instituted only after consultation with the Knoll medical monitor.

If there was a documented lack of efficacy (ie, failure to achieve ACR20 response compared with baseline) the following could occur:
- A single increase in dosage of background DMARD and/or steroid therapy (provided the prednisone dose remained ≤10 mg/day).
- On or after 3 months, initiation of treatment with another DMARD therapy listed above.
- Further dose adjustments in background anti-rheumatic drugs with prior approval from the Knoll medical monitor.

Patients receiving azathioprine and/or cyclosporine were required to discontinue these therapies and enter a 28-day washout period before the screening visit. These therapies could not be used during the study.

Concomitant therapies were defined as those therapies used at baseline and continued during the study, or those initiated during the study. Concomitant therapies are presented by preferred term and treatment group for all randomized patients.

Concomitant therapies were divided into "RA-specific" and "non-RA-specific" according to the indications specified by the investigators on the CRF. All patients were on some form of RA-specific concomitant medication (DMARD or non-DMARD) during the study.

### RA-specific concomitant DMARD therapies:

Concomitant DMARD therapies are presented by preferred term and treatment group for all randomized patients in Table 1.

**Table 1 Number (%) of patients with the most frequently reported^{a} RA-specific concomitant DMARD therapies by randomized treatment group (all randomized patients)**

| Concomitant thotapies^{b} | D2E7 (N=318) | Placebo (N=318) |
|---|---|---|
| At least one relevant RA-specific concomitant DMARD treatment | 261 (82.1) | 270 (84.9) |
| Methotrexate | 178 (56.0) | 199 (62.6) |
| Antimalarials | 76 (23.6) | 82 (26.8) |
| Leflunomide | 42 (13.2) | 46 (14.5) |
| Sulfasalazine | 29 (9.1) | 33 (10.4) |
| Gold | 19 (6.0) | 18 (5.7) |

| | | |
|---|---|---|
| ^{a} Occurring in ≥5% of patients overall, ^{b} Patients may appear in more than one class. | | |

A majority of randomized patients (531 [83.5%] of 636) used one or more concomitant DMARD therapies during the study (261 [82.1%] of 318 D2E7-treated patients and 270 [84.9%] of 318 placebo-treated patients). A total of 105 (16.5% of 636) patients did not use DMARDs (57 [17.9%] of 318 D2E7-treated patients and 48 [15.1%] of 318 placebo-treated patients), 356 (56.0% of 636) patients used one DMARD (184 [57.9%] of 318 D2E7-treated patients and 172 [54.1%] of 318 placebo-treated patients), and 175 patients (27.5% of 636) used between two and four different DMARDs (77 [24.2%] of 318 D2E7-treated patients and 98 [30.8%] of 318 placebo-treated patients).

The D2E7-treatment group used a mean of 1.10 different DMARDs while the placebo-treated group used a mean of 1.21 different DMARDs during the study.

The most frequently reported concomitant DMARD therapy was methotrexate, which was used by 377 (59.3%) of 636 patients (178 [56.0%] of 318 D2E7-treated patients and 199 [62.6%] of 318 placebo-treated patients). The next three most commonly used concomitant DMARD therapies were antimalarials (ie, chloroquine, hydroxychloroquine) (24.7% of 636 patients: 23.6%a of 318 D2E7-treated patients and 25.8% of 318 placebo-treated patients), leflunomide (13.8% of 636 patients: 13.2% of 318 D2E7-treated patients and 14.5% of 318 placebo-treated patients), and sulfasalazine (9.7% of 636 patients: 9.1% of 318 D2E7-treated patients and 10.4% of 318 placebo-treated patients). There were no relevant differences between D2E7- and placebo-treated patients in the frequency of use of RA-specific concomitant DMARD therapies.

### RA-specific non-DMARD concomitant therapies:

RA-specific non-DMARD concomitant therapies are presented by preferred term and treatment group for all randomized patients in Table 2.

**Table 2 Number (%) of patients with the most frequency reported^{a} RA-specific non-DMARD concomitant therapies by randomized treatment group (all randomized patients)**

| Concomitant theraples^{b} | D2E7 (N=318) | Placebo (N=318) |
|---|---|---|
| At least one relevant RA-specitic non-DMARD concomitant treatment | 318 (99.1) | 304 (95.6) |
| Prednisone | 141 (44.3) | 146 (45.9) |
| Follo acid | 88 (27.7) | 86 (27.0) |
| Celecoxib | 77 (24.2) | 83 (26.1) |
| Rofecoxib | 45 (14.2) | 39 (12.3) |
| Paracetamol | 39 (12.3) | 49 (15.4) |
| Naproxen | 33 (10.4) | 40 (12.6) |
| Ibuprofen | 28 (8.8) | 23 (7.2) |
| Mothylprednisolone | 21 (6.6) | 27 (8.5) |
| Tramadol | 17 (5.3) | 14 (4.4) |
| DI-gesic | 17 (5.3) | 11 (3.5) |
| Diclofenac | 15 (4.7) | 18 (5.7) |
| Vicodin | 14 (4.4) | 21 (6.6) |
| Triamcinolone | 13 (4.1) | 37 (11.6) |
| Lidocaine | 10 (3.1) | 22 (6.9) |

| | | |
|---|---|---|
| ^{a} Occurring In ≥5% of patients overall. ^{b} Patients may appear In more than one class. | | |

A total of 619 (97.3%) of 636 randomized patients (315 [99.1%] of 318 D2E7-treated patients and 304 [95.6%] of 318 placebo-treated patients) used one or more RA-specific concomitant non-DMARD therapies during the study.

The most frequently reported RA-specific non-DMARD concomitant therapy was prednisone, which was used by 287 (45.1%) of 636 patients (141 [44.3%] of 318 D2E7-treated patients and 146 [45.9%] of 318 placebo-treated patients). The next most commonly used non-DMARD concomitant therapy was folic acid (27.4% of 636 patients: 27.7% of 318 D2E7-treated patients and 27.0% of 318 placebo-treated patients). (Folic acid was classified as a RA-specific non-DMARD therapy or as a non-RA-specific therapy depending upon the indication that was recorded in the CRF. For all indications other than RA, folic acid was classified a non-RA-specific therapy. Celecoxib (25.2% of 636 patients: 24.2% of 318 D2E7-treated patients and 26.1% of 318 placebo-treated patients) and rofecoxib (13.2% of 636 patients: 14.2% of 318 D2E7-treated patients and 12.3% of 318 placebo-treated patients) were the next most frequently used RA-specific non-DMARD concomitant therapies. There were no relevant differences between D2E7-and placebo-treated patients in the frequency of use of RA-specific non-DMARD concomitant therapies.

### Non-RA-specific concomitant therapies:

Non-RA-specific concomitant therapies are presented by preferred term and treatment group for all randomized patients.

A total of 614 (96.5%) of 636 randomized patients (306 [96.2%] of 318 D2E7-treated patients and 308 [96.9%] of 318 placebo-treated patients) used one or more non-RA-specific concomitant therapies.

The most frequently reported non-RA-specific concomitant therapy was multivitamins, which was used by 166 (26.1%) of 636 patients (78 [24.5%] of 318 D2E7-treated patients and 88 [27.7%] of 318 placebo-treated patients). The next three most commonly used concomitant therapies were calcium (24.4% of 636 patients: 23.6% of 318 D2E7-treated patients and 25.2% of 318 placebo-treated patients), folic acid (22.2% of 636 patients: 21.4% of 318 D2E7-treated patients and 23.0% of 318 placebo-treated patients) and influenza virus vaccine polyvalent (14.2% of 636 patients: 14.2% of 318 D2E7-treated patients and 14.2% of 318 placebo-treated patients). (Folic acid was classified as a RA-specific non-DMARD therapy or as a non-RA-specific therapy depending upon the indication that was recorded in the CRF. For all indications other than RA, folic acid was classified a non-RA-specific therapy.) There were no relevant differences between D2E7- and placebo-treated patients in the frequency of use of non-RA-specific concomitant therapies.

Efficacy endpoints included: ACR20 response at Week 24; ACR50 and ACR70 at Week 24; AUC (area under the curve) of ACR20, ACR50 and ACR70 from baseline to Week 24; time to first response for ACR20, ACR50 and ACR70; numeric ACR (ACR-N); change from baseline to Week 24 in components of the ACR response criteria (tender joint count [TJC], swollen joint count [SJC], patient assessment of pain, patient and physician global assessments of disease activity, HAQ and C-reactive protein [CRP]); change from baseline to Week 24' in physical function as measured by SF-36, health utilities index (HUI), functional assessment of chronic illness therapy (FACIT) fatigue scale; Euro-QoL questionnaire; multidimensional assessment of fatigue (MAF) scale; subject summary survey; incidence and time to dose increases in background DMARD and/or steroid therapy; institution of new DMARD therapy for lack of clinical response; rheumatoid factor (RF); and morning stiffness.

Adverse events were summarized by frequency, percentage, as well as the rate per 100 patient-years. Statistical comparisons were made between the D2E7 and placebo groups using Pearson's chi-squared (χ²) tests. Changes in physical examination, laboratory parameters and chest x-ray were described by statistical characteristics, as well as frequency of abnormal values. Shift tables were also provided. Vital signs were described by statistical characteristics. The ACR20 response at Week 24 (change from baseline) was defined as the primary efficacy variable. The ACR20 response rates were compared between the D2E7 and placebo groups using Pearson's χ² test with a two-sided level of significance of α=0.05. All other efficacy variables were summarized descriptively (statistical characteristics, frequencies, percentages, confidence intervals) and analyzed by exploratory two-sided statistical tests. For categorical data the Pearson's χ² test was used, for continuous data an analysis of covariance (ANCOVA) model was used that included the treatment group as a factor and the respective baseline value as a covariate. Demographic and baseline characteristics were compared between the D2E7 and placebo groups using the Wilcoxon rank sum test for continuous variables and the Pearson's χ² test for discrete variables.

### Efficacy results:

The primary efficacy endpoint, ACR20 response, was associated with a statistically significantly greater improvement (53.0% for D2E7 *vs*. 34.9% for placebo) in patients who had D2E7 added to their current anti-rheumatic therapies compared to placebo.

| | | |
|---|---|---|
| ACR | D2E7 | Placibo |
| | (N=315) | (N=315) |
| | N (%) | N (%) |
| | | |
| ACR20 | 167 (53.0) | 110 (34.9) |
| ACR50 | 92 (29.2) | 35 (11.1) |
| ACR70 | 47 (14.9) | 10 (3.2) |

Treatment with D2E7 was associated with a statistically significant improvement on the secondary efficacy endpoints of ACR50, ACR70, ACR-N, tender joint count, swollen joint count, patient and physician global assessment of disease activity, patient assessment of pain, disability index of the HAQ, C-reactive protein, morning stiffness, duration of morning stiffness, FACIT fatigue scale, 9 of 10 domains of the SF-36 and 7 of 16 domains of the HUI.

D2E7 also demonstrated improvement in therapeutic response over placebo for the endpoints of time to response for ACR20, ACR50, and ACR70; AUC for ACR20, ACR50, ACR70 and ACR-N; Euro-QoL, RF, and MAP scale.

Subgroup analyses demonstrated greater improvement for D2E7 patients receiving concomitant methotrexate, antimalarial treatments, sulfasalazine, other DMARDs, or the number of concomitant DMARDs (ie, 0, 1., 2, or ≥3) for the parameters of ACR20, ACR50, ACR70, ACR-N, and AUC of ACR20, ACR50, ACR70, and ACR-N scores compared to placebo patients. Concomitant leflunomide was similar to placebo for all parameters tested.

**Table 3 Subgroup analysis at Week 24 for ACR20**

| Concomitant medication | ACR20 | | | |
|---|---|---|---|---|
| | D2E7 | | Placebo | |
| | Total N | % Response | Total N | % Response |
| Methotrexate | 178 | 56.7 | 199 | 35.2 |
| Antimalarial | 76 | 50.7 | 82 | 32.9 |
| Leflunomide | 42 | 33.3 | 46 | 37.0 |
| Sultasalazine | 29 | 58.6 | 33 | 24.2 |
| Other DMARDs | 25 | 52.0 | 25 | 44.0 |
| No DMARD | 54 | 50.0 | 46 | 33.3 |
| One DMARD | 184 | 55.4 | 172 | 37.8 |
| Two DMARDs | 66 | 50.0 | 84 | 29.8 |
| Three or more DMARDs | 11 | 45.5 | 14 | 35.7 |

| | | | | |
|---|---|---|---|---|
| Antimalarial (eg, HCG, chloroquine) | | | | |

**Table 4: ACR50, ACR70, and ACFt-N response rates: patients responding at Week24 by randomized treatment group**

| | D2E7 | Placebo |
|---|---|---|
| | (N=315) | (N=316) |
| Time point | N(%) | N (%) |
| ACR50 | | |
| Week 24 | 92 (29.2)^{a} | 35 (11.1) |
| LOCF Week 24 | 95 (30.2) | 35 (11.1) |

| ACR70 | | |
|---|---|---|
| Week 24 | 47 (14.9)^{a} | 10 (3.2) |
| LOCF Week 24 | 48 (15.2) | 10 (3.2) |

| | Mean ± SD | Mean ± SD |
|---|---|---|
| ACR-N | | |
| Week 24 | 20.3 ± 65.2 | 0.8 ± 47.0 |
| LOAF Week 24 | 21.2 ± 65.3 | 1.0 ± 47.0 |

| | | |
|---|---|---|
| ^{a} Statistically significantly different from placebo (p<0,001). | | |

**Table 5 Subgroup analysis at Week 24 for ACR50, ACR70, and ACR-N**

| | ACR50 | | | | ACR70 | | | | ACR-N | | | |
|---|---|---|---|---|---|---|---|---|---|---|---|---|
| | D2E7 | | Placebo | | D2E7 | | Placebo | | | D2E7 | | Placebo |
| Concomitant | (N=315) | | (N=315) | | (N=315) | | (N=315) | | | (N=315) | | (N=315) |
| medication | N | % | N | % | N | % | N | % | N | Mean ± SD | N | Mean ± SD |
| Methotrexate | 178 | 30.9 | 199 | 12.1 | 178 | 16.2 | 199 | 2.0 | 178 | 36.5 ± 42.3 | 199 | 3.2 ± 43.0 |
| Antimalarial | 75 | 29.3 | 82 | 13.4 | 75 | 12.0 | 82 | 2.4 | 76 | 20.0 ± 45.5 | 82 | 3.4 ± 43.4 |
| Leflunomide | 42 | 14.3 | 46 | 10.9 | 42 | 0.0 | 46 | 8.7 | 42 | -0.5 ± 77.5 | 46 | -4,5 ± 67.3 |
| Sulfasalazine | 29 | 34.5 | 33 | 12.1 | 29 | 17.2 | 33 | 6.1 | 29 | 21.4 ± 53.9 | 33 | -1.7 ± 41.5 |
| Other | | | | | | | | | | | | |
| DMARDs | 25 | 24.0 | 26 | 8.0 | 25 | 16.0 | 25 | 4.0 | 25 | 26.3 ± 33.9 | 25 | 6.7 ± 49.1 |
| No DMARD | 54 | 27.8 | 45 | 6.7 | 54 | 18.5 | 45 | 0.0 | 54 | 12.8 ± 68.2 | 45 | -7.5 ± 54.6 |
| One DMARD | 184 | 31.0 | 172 | 12.2 | 184 | 16.3 | 172 | 5.2 | 184 | 22.8 ± 56.0 | 172 | 4.2 ± 45.6 |
| Two | | | | | | | | | | | | |
| DMARDs | 66 | 27.3 | 84 | 10.7 | 66 | 9.1 | 84 | 0.0 | 66 | 19.5 ± 42.3 | 84 | -4.3 ± 46.9 |
| Three or | | | | | | | | | | | | |
| more | | | | | | | | | | | | |
| DMARDs | 11 | 18.2 | 14 | 14.3 | 11 | 9.1 | 14 | 7.1 | 11 | 20.1 ± 37.2 | 14 | 16.5 ± 30.5 |

| | | | | | | | | | | | | |
|---|---|---|---|---|---|---|---|---|---|---|---|---|
| Antimalarial (eg, HCG, chloroquine). | | | | | | | | | | | | |

**Table 6 Time to response according to ACR20, ACR50, ACR70: number (%) of patients responding for the first time by randomized treatment group (full analysis set, excluding Site #7)**

| | ACR20 | | ACR50 | | ACR70 | |
|---|---|---|---|---|---|---|
| | D2E7 (N=315) | Placebo (N=315) | D2E7 (N=315) | Placebo (N=315) | D2E7 (N=315) | Placebo (N=315) |
| Time to response | N (%) | N (%) | N (%) | N (%) | N (%) | N (%) |
| Week 2 | 106 (33.7) | 27 (8.6) | 31 (9.8) | 3 (1.0) | 9 (2.9) | 0 (0.0) |
| Week 4 | 51 (16.2) | 43 (13.7) | 31 (9.8) | 7 (2.2) | 12 (3.8) | 1 (0.3) |
| Week 8 | 52 (16.5) | 40 (12.7) | 34 (10.8) | 12 (3.8) | 12 (3.8) | 2 (0.6) |
| Week 12 | 17 (5.4) | 32 (10.2) | 20 (6.3) | 13 (4.1) | 18 (5.7) | 5 (1.6) |
| Week 16 | 13 (4.1) | 20 (6.3) | 20 (6.3) | 15 (4.8) | 18 (5.7) | 7 (2.2) |
| Week 20 | 12 (3.8) | 15 (4.8) | 9 (2.9) | 13 (4.1) | 7 (2.2) | 6 (1.9) |
| Week 24 | 3 (1.0) | 10 (3.2) | 10 (3.2) | 11 (3.5) | 8 (2.5) | 3 (1.0) |
| Non-responder | 61 (19.4) | 128 (40.6) | 160 (60.8) | 241 (76.5) | 231 (73.3) | 291 (92.4) |

### Conclusion:

D2E7 was generally well-tolerated when added to patients' existing DMARD therapies (eg, methotrexate, antimalarials [chloroquine/hydroxychloroquine], leflunomide, and sulfasalazine). Its addition was not associated with any major changes in the incidence or profile of adverse events. Additionally, the adverse event profile was not affected by the total number of concomitant DMARDs (ie, 0, 1, 2, or ≥3) that were used by patients.

Overall, D2E7 at a dose of 40 mg was demonstrated to be safe when used alone or in combination with other DMARDs.

D2E7 treatment was associated with a significant improvement in RA in patients whose disease was inadequately treated with their existing DMARD therapies. When used in combination with methotrexate, antimalarial treatments, sulfasalazine, other DMARDs, or the number of concomitant DMARDs (ie, 0, 1, 2, or ≥3), D2E7 was associated with a higher response compared to placebo. Finally, improvements in response rates were generally independent of the type or number of DMARDs used.

### Further Embodiments of the Invention

### Embodiment A:

The present invention provides a method for treating a disorder in a human subject which disease is treatable with a TNFα antibody or antigen binding fragment thereof, comprising administering a composition to the human subject in need thereof, on a biweekly dosing regimen such that the disorder is treated, said composition comprising an anti-TNFα antibody or an antigen binding portion thereof and administering one or more other drug(s).

### Embodiment B:

In this method according to Embodiment A, the administration of the composition comprising an anti-TNFα antibody or an antigen binding portion thereof is by subcutaneous injection.

### Embodiment C:

In this method according to Embodiment A, said anti-TNFα antibody or an antigen binding portion thereof is a human anti-TNFα antibody.

### Embodiment D:

In this method according to Embodiment C, said human antibody is D2E7.

### Embodiment E;

In this method according to Embodiment D, 40 mg of D2E7 is administered.

### Embodiment F:

In this method according to Embodiment E, the disease is an autoimmune disease.

### Embodiment G:

In this method according to Embodiment F, the autoimmune disease is rheumatoid arthritis.

### Embodiment H:

In this method according to Embodiment G, the other drug is a Disease Modifying Anti-Rheumatic Drug (DMARD), a Nonsteroidal Antiinflammatory Drug (NSAID), a steroid or any combination thereof.

### Embodiment I:

In this method according to Embodiment H, the DMARD is hydroxychloroquine, leflunomide, methotrexate, parenteral gold, oral gold, sulfasalazine or any combination thereof.

### Embodiment J:

In this method according to Embodiment H, the NSAID is Prednisone, Folic acid, Celecoxib, Rofecoxib, Paracetamol, Naproxen, Ibuprofen, Methylprednisolone, Tramadol, Di-gesic, Diclofenac, Vicodin, Triamcinolone, Lidocaine or any combination thereof.

### Embodiment K:

In this method according to Embodiment G, the other drug is multivitamins, calcium, folic acid, influenza virus vaccine polyvalent or any combination thereof.

### Embodiment L:

The present invention also provides a pharmaceutical composition comprising 40 mg of D2E7, one or more other drug(s) and a pharmaceutically acceptable carrier.

### Embodiment M:

In this pharmaceutical composition according to Embodiment L, the other drug is a Disease Modifying Anti-Rheumatic Drug (DMARD), a Nonsteroidal Antiinflammatory Drug (NSAID), a steroid or any combination thereof.

### Embodiment N:

In this pharmaceutical composition according to Embodiment M, the DMARD is hydroxychloroquine, leflunomide, methotrexate, parenteral gold, oral gold, sulfasalazine or any combination thereof.

### Embodiment O:

In this pharmaceutical composition according to Embodiment M, the NSAID is Prednisone, Folic acid, Celecoxib, Rofecoxib, Paracetamol, Naproxen, Ibuprofen, Methylprednisolone, Tramadol, Di-gesic, Diclofenac, Vicodin, Triamcinolone, Lidocaine or any combination thereof.

### Embodiment P:

In this pharmaceutical composition according to Embodiment L, the other drug is multivitamins, calcium, folic acid, influenza virus vaccine polyvalent or any combination thereof.

### Embodiment Q:

The present invention also provides a kit comprising a formulation comprising:
a) a pharmaceutical composition comprising an anti-TNFα antibody and a pharmaceutically acceptable carrier;
b) one or more pharmaceutical compositions, each composition comprising one or more other drug(s) and a pharmaceutically acceptable carrier; and
c) instructions for biweekly dosing of the pharmaceutical composition for the treatment of a disorder in which an anti-TNFα antibody or a binding portion thereof is effective in treating.

### Embodiment R:

In this kit according to Embodiment Q, the composition comprising the antibody is 40 mg of D2E7 and instructions pertain to treating rheumatoid arthritis.

### Embodiment S:

In this kit according to Embodiment R, the other composition or compositions comprise a Disease Modifying Anti-Rheumatic Drug (DMARD), a Nonsteroidal Antiinflammatory Drug (NSAID), a steroid or any combination thereof.

### Embodiment T:

In this kit according to Embodiment S, the DMARD is hydroxychloroquine, leflunomide, methotrexate, parenteral gold, oral gold, sulfasalazine or any combination thereof.

### Embodiment U:

In this kit according to Embodiment S, the NSAID is Prednisone, Folic acid, Celecoxib, Rofecoxib, Paracetamol, Naproxen, Ibuprofen, Methylprednisolone, Tramadol, Di-gesic, Diclofenac, Vicodin, Triamcinolone, Lidocaine or any combination thereof.

### Embodiment V:

In this kit according to Embodiment R, the other drug is multivitamins, calcium, folic acid, influenza virus vaccine polyvalent or any combination thereof.

### EQUIVALENTS

Those skilled in the art will recognize, or be able to ascertain using no more than routine experimentation, many equivalents to the specific embodiments of the invention described herein. Such equivalents are intended to be encompassed by the following claims.

## Claims

1. A composition comprising an isolated human anti-TNFα antibody, or an antigen-binding portion thereof for treating a disorder in a human subject in which TNFα is detrimental, wherein the composition is to be administered in combination with one or more other drug(s) selected from the group consisting of prednisone, folic acid, celecoxib, rofecoxib, paracetamol, naproxen, tramadol, ibuprofen, Di-gesic, diclofenac, methylprednisolone, tramadol, vicodin, triamcinolone, lidocaine, multivitamins, calcium, and influenza virus vaccine polyvalent.

2. A composition comprising an isolated human anti-TNFα antibody, or an antigen-binding portion thereof, for use in the treatment of a disorder in a human subject in which TNFα is detrimental, wherein the composition is to be administered in a combination therapy with:
(a) one or more RA-specific non-DMARD drugs; and,
(b) one or more non-RA specific drugs; and, optionally,
(c) one or more RA-specific DMARDs.

3. The composition of claim 2, wherein:
(a) said one or more RA-specific non-DMARD drug is selected from the group consisting of: prednisone, folic acid, celecoxib, rofecoxib, paracetamol, naproxen, ibuprofen, methylprednisolone, tramadol, Di-gesic, diclofenac, vicodin, and triamcinolone;
(b) said one or more non-RA specific drug is selected from the group consisting of: multivitamins, calcium, and influenza virus vaccine polyvalent, or,
(c) said one or more RA-specific DMARD is selected from the group consisting of: methotrexate, antimalarial drug (such as chloroquine or hydroxychloroquine), saulfasalazine, parenteral gold, and oral gold.

4. The composition of any one of claims 1-3, wherein said antibody, or antigen-binding portion thereof, dissociates from human TNFα with a K_{d} of 1 x 10⁻⁸ M or less and a *k_{off}* rate constant of 1 x 10⁻³ s⁻¹ or less, both determined by surface plasmon resonance, and neutralizes human TNFα cytotoxicity in a standard *in vitro* L929 assay with an IC₅₀ of 1 x 10⁻⁷ M or less.

5. The composition of any one of claims 1-4, wherein said antibody, or antigen-binding portion thereof, has the following characteristics:
a) dissociates from human TNFα with a *k_{off}* rate constant of 1 x 10⁻³ s⁻¹ or less, as determined by surface plasmon resonance;
b) has a light chain CDR3 domain comprising the amino acid sequence of SEQ ID NO: 3, or modified from SEQ ID NO: 3 by a single alanine substitution at position 1, 4, 5, 7 or 8 or by one to five conservative amino acid substitutions at positions 1, 3, 4, 6, 7, 8 and/or 9; and
c) has a heavy chain CDR3 domain comprising the amino acid sequence of SEQ ID NO: 4, or modified from SEQ ID NO: 4 by a single alanine substitution at position 2, 3, 4, 5, 6, 8, 9, 10 or 11 or by one to five conservative amino acid substitutions at positions 2, 3, 4, 5, 6, 8, 9, 10, 11 and/or 12.

6. The composition of any one of claims 1-5, wherein said antibody, or antigen-binding portion, thereof has a light chain variable region (LCVR) comprising the amino acid sequence of SEQ ID NO: 1 and a heavy chain variable region (HCVR) comprising the amino acid sequence of SEQ ID NO: 2.

7. The composition of any one of claims 1-6, wherein said antibody is D2E7.

8. The composition of any one of claims 1-7, wherein the administration of the composition comprising said antibody, or antigen-binding portion thereof, is by subcutaneous injection, as a biweekly dosing regimen, or a combination thereof.

9. The composition of any one of claims 1-8, having a dose of 10-100 mg of said antibody, or antigen-binding portion thereof, and, optionally, about 40 mg.

10. The composition of any one of claims 1-9, wherein the disorder is selected from the group consisting of an autoimmune disease, sepsis, an intestinal disorder, an infectious disease, transplant rejection or graft-versus-host disease, a malignancy, a pulmonary disorder, a cardiac disorder, and a neurological disorder.

11. The composition of claim 10, wherein the autoimmune disease is selected from the group consisting of rheumatoid arthritis, rheumatoid spondylitis, osteoarthritis and gouty arthritis, allergy, multiple sclerosis, autoimmune diabetes, autoimmune uveitis, and nephrotic syndrome.

12. A kit comprising:
a) a pharmaceutical composition comprising an isolated human anti-TNFα antibody, or an antigen-binding portion thereof, which inhibits TNFα activity, and a pharmaceutically acceptable carrier; and
b) one or more pharmaceutical compositions, each comprising one or more other drug(s) and a pharmaceutically acceptable carrier, wherein said one or more other drug(s) is selected from the group consisting of prednisone, folic acid, celecoxib, rofecoxib, paracetamol, naproxen, tramadol, ibuprofen, Di-gesic, diclofenac, methylprednisolone, tramadol, vicodin, triamcinolone, lidocaine, multivitamins, calcium, and influenza virus vaccine polyvalent, or any combination thereof; and, optionally,
c) instructions for biweekly dosing and/or subcutaneous administration of the pharmaceutical composition comprising the anti-TNFα antibody or antigen-binding portion thereof, for the treatment of a disorder in which TNFα is detrimental.

13. The kit of claim 12, wherein said antibody, or antigen-binding portion thereof, is selected from the group consisting of
a) an antibody, or antigen-binding portion thereof, that dissociates from human TNFα with a K_{d} of 1 x 10⁻⁸ M or less and a *k_{off}* rate constant of 1 x 10⁻³ s⁻¹ or less, both determined by surface plasmon resonance, and neutralizes human TNFα cytotoxicity in a standard *in vitro* L929 assay with an IC₅₀ of 1 x 10⁻⁷ M or less;
b) an antibody, or antigen-binding portion thereof, having the following characteristics:
i) dissociates from human TNFα with a *k_{off}* rate constant of 1 x 10⁻³ s⁻¹ or less, as determined by surface plasmon resonance;
ii) has a light chain CDR3 domain comprising the amino acid sequence of SEQ ID NO: 3, or modified from SEQ ID NO: 3 by a single alanine substitution at position 1, 4, 5, 7 or 8 or by one to five conservative amino acid substitutions at positions 1, 3, 4, 6, 7, 8 and/or 9; and
iii) has a heavy chain CDR3 domain comprising the amino acid sequence of SEQ ID NO: 4, or modified from SEQ ID NO: 4 by a single alanine substitution at position 2, 3, 4, 5, 6, 8, 9, 10 or 11 or by one to five conservative amino acid substitutions at positions 2, 3, 4, 5, 6, 8, 9, 10, 11 and/or 12;
c) an antibody, or antigen-binding portion thereof, comprising a light chain variable region (LCVR) comprising the amino acid sequence of SEQ ID NO: 1, and a heavy chain variable region (HCVR) comprising the amino acid sequence of SEQ ID NO: 2; and
d) an antibody which is D2E7.

14. The kit of claim 12 or 13, wherein said pharmaceutical composition comprising the anti-TNFα antibody or antigen-binding portion thereof, includes dose of between 10-100 mg of the antibody or antigen-binding portion thereof, and, optionally, a dose of about 40 mg.

15. The kit of any one of claims 12-14, wherein the disorder is selected from the group consisting of an autoimmune disease, sepsis, an intestinal disorder, an infectious disease, transplant rejection or graft-versus-host disease, a malignancy, a pulmonary disorder, a cardiac disorder, and a neurological disorder.
